(19) Europäisches Patentamt
European Patent Office
Office européen des brevets

(11) **EP 3 187 578 A1**

(12) **EUROPEAN PATENT APPLICATION**
published in accordance with Art. 153(4) EPC

(43) Date of publication:
**05.07.2017 Bulletin 2017/27**

(51) Int Cl.:
*C12N 1/02* (2006.01)    *C12M 1/00* (2006.01)

(21) Application number: **15835573.5**

(86) International application number:
**PCT/JP2015/074457**

(22) Date of filing: **28.08.2015**

(87) International publication number:
**WO 2016/031971 (03.03.2016 Gazette 2016/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **29.08.2014 US 201462043454 P**

(71) Applicant: **Hitachi Chemical Company, Ltd.**
**Chiyoda-ku**
**Tokyo 100-6606 (JP)**

(72) Inventors:
• **YAGI Satomi**
  **Tokyo 100-6606 (JP)**
• **TAKAI Kenji**
  **Tokyo 100-6606 (JP)**
• **KIKUHARA Yoshihito**
  **Tokyo 100-6606 (JP)**
• **TSAI Anthony H.**
  **Irvine, CA 92617 (US)**

(74) Representative: **Hoffmann Eitle**
**Patent- und Rechtsanwälte PartmbB**
**Arabellastraße 30**
**81925 München (DE)**

(54) **CELL TRAPPING METHOD, METHOD FOR PRODUCING SPECIFIC CELL-TRAPPING DEVICE, AND METHOD FOR PRODUCING SPECIFIC CELL-CONTAINING SOLUTION**

(57) Provided is a cell trapping method for selectively trapping a specific cell included in a cell-containing solution at a filter surface by filtering a liquid using a cell trapping device, the cell trapping device includes a housing which has an introduction region for introducing a liquid selected from the cell-containing solution containing two or more types of cells and a treating solution for treating the cells in the cell-containing solution into an interior, and a discharge flow path for discharging the liquid to the exterior; and a filter which is provided on a flow path between the introduction region and the discharge flow path and in which a plurality of through holes for the liquid to flow through is formed in the thickness direction, and the method includes a step of draining a liquid for n (n is a natural number) times, in which from the first step of draining the liquid to the n-th step of draining the liquid, a liquid surface of the liquid in the introduction region on the filter is maintained at a predetermined liquid surface height, and when the n-th step of draining the liquid is completed, the discharging of the liquid from the cell trapping device is stopped in a state where the liquid surface is at a predetermined height in the filter.

EP 3 187 578 A1

## Description

### Technical Field

The present invention relates to a method for trapping a specific cell, by which only a specific cell is trapped and concentrated without imparting damage to the cell selectively trapped when only the specific cell is trapped and detected from blood and a cell-containing solution containing two or more types of cells by means of a filtration system, a method for producing a specific cell-trapping device after the trapped specific cell is trapped, and a method for producing a solution containing the trapped specific cell.

### Background Art

In recent years, in the field of blood, a technology of trapping and detecting only a specific cell from a solution has been greatly developed. Many studies have been conducted for a technology of removing only a white blood cell at the time of blood transfusion or a technology of extracting a specific white blood cell in immunotherapy. Among these, a technology of detecting a circulating tumor cell (hereinafter, CTC) has been particularly highlighted. Lots of studies have been conducted for CTC because of clinical significance and in recent years, particularly, it is expected that the CTC is applied to screening of an anticancer drug or a diagnosis technology of detecting cancer in an early time.

In recent years, a method for effectively detecting a small amount of the CTC by using a resin filter using parylene has been suggested (Patent Literature 1).

Alternatively, a method has been suggested, which enhances the strength of a filter by using a filter using a metal instead of the resin and separates a cancer cell and a white blood cell by using a difference in deformability therebetween(Patent Literature 2).

### Citation List

#### Patent Literatures

[Patent Literature 1] International Publicatio No. 2010/135603
[Patent Literature 2] Japanese Unexamined Patent Publication No. 2013-42689

### Summary of Invention

#### Technical Problem

In a step of extracting a specific cell such as a rare cell from blood and a cell-containing solution containing two or more types of cells using a filtration technology, it is desired to trap and concentrate only the specific cell without imparting damage with respect to the selectively trapped cell.

For example, in the conventionally known filter method, a white blood cell, a red blood cell, or a platelet in the blood can be effectively removed, but since the white blood cell has a size close to a cancer cell, it is not possible to remove all of the cells.

The filter method is a method which separates the white blood cell and the cancer cell using a difference in deformability and size between the white blood cell and the cancer cell. In this method, a study on a material, a pore shape, a pore diameter, an aperture ratio, and a flow rate of the filter considerably enables separation of the white blood cell and the cancer cell.

However, after the white blood cell and the cancer cell are separated to each other by conducting filtration, it is not easy to extract the trapped cancer cell from the filter and the cell may be damaged at the time of extraction.

An object of the present invention is to provide a cell trapping method, by which a specific cell is extracted without imparting damage to the trapped cell after the specific cell is trapped by filtration, a method for producing a specific cell-trapping device for extraction, and a method for producing a specific cell-containing solution containing the trapped cell.

### Solution to Problem

As a result of repeated studies, the present inventors have enabled a cell to be extracted without imparting damage to the cell, by continuously performing a liquid substitution without taking the liquid out at all in a cartridge (cell trapping device) during the time from the cell is trapped by filtration and the cell is analyzed and detected. According to method of the present invention, it is possible to prevent the cell from being pinched within a pore and the cell from being deformed, and accordingly it is expected that the cell is collected in a highly effective manner and the cell is collected with less damage.

When only the specific cell is trapped from a cell-containing solution by filtration, the treatment is performed in a state where a liquid surface remains at the height of the average particle diameter or greater of the target trapped cell from a filter surface, thereby preventing the cell from being pinched within a pore and the cell from being deformed. Thus, it is possible to extract the cell without imparting damage to the cell.

That is, provided is a cell trapping method according to one aspect of the present invention for selectively trapping a specific cell included in a cell-containing solution at a filter surface by filtering a liquid using a cell trapping device, the cell trapping device including a housing which has an introduction region having a predetermined volume and for introducing a liquid selected from the cell-containing solution containing two or more types of cells and a treating solution for treating the cells in the cell-containing solution into an interior, and a discharge flow path for discharging the liquid to the exterior; and a filter which is provided on a flow path between the introduction region and the discharge flow path and in which a plurality of through holes for the liquid to flow through is formed in the thickness direction, the method includes: a step of draining a liquid to the cell trapping device for n (n is a natural number) times, in which from the first step of draining the liquid to the n-th step of draining the liquid, a liquid surface of the liquid in the introduction region on the filter is maintained at a predetermined liquid surface height, and when the n-th step of draining the liquid is completed, the discharging of the liquid from the cell trapping device is stopped in a state where the liquid surface is at a predetermined height in the filter.

Here, the housing may include an upper lid disposed above the introduction region and an introduction flow path of the liquid.

Before the first step of draining a liquid, a washing solution may fill in the upstream side than the filter including the interior of the introduction region of the housing in advance.

The step of draining a liquid may include at least one step of a step of draining a fixing solution, a step of draining a permeating solution, a step of draining a staining solution, and a step of draining a washing solution; and the step of draining a cell-containing solution.

The n may be 1 and the step of draining a liquid may be the step of draining a cell-containing solution.

In the step of draining a liquid, the liquid to be drained for the first time may be a cell-containing solution, and the liquid to be drained for the second time may be a washing solution.

In the step of draining a liquid, at least part of the trapping target cell may be immersed in the liquid in the introduction region on the filter.

In the step of draining a liquid, the height of the liquid surface of the liquid in the introduction region on the filter may be equal to or greater than the size of the trapping target cell.

In the step of draining a liquid, the height of the liquid surface of the liquid in the introduction region on the filter may be 50 $\mu$m or greater.

A solvent of the cell-containing solution may include an aqueous solution containing whole blood, serum, or plasma of a mammal, or a protein originated therefrom.

The filter may be processed by using a polymeric material or a metal.

In the step of draining a liquid, a rate of introducing the liquid to the cell trapping device may be 50 to 1000 $\mu$L/min.

In addition, provided is a method for producing a specific cell-trapping device according to another aspect of the present invention for producing a specific cell-trapping device which selectively traps a specific cell included in a cell-containing solution at a filter surface by filtering a liquid using a cell trapping device, the cell trapping device including a housing which has an introduction region having a predetermined volume and for introducing a liquid selected from the cell-containing solution containing two or more types of cells and a treating solution for treating the cells in the cell-containing solution into an interior, and a discharge flow path for discharging the liquid to the exterior; and a filter which is provided on a flow path between the introduction region and the discharge flow path and in which a plurality of through holes for the liquid to flow through is formed in the thickness direction, the method includes: a step of draining a liquid to the cell trapping device for n (n is a natural number) times, in which from the first step of draining the liquid to the n-th step of draining the liquid, a liquid surface of the liquid in the introduction region on the filter is maintained at a predetermined liquid surface height, and when the n-th step of draining the liquid is completed, the discharging of the liquid from the cell trapping device is stopped in a state where the liquid surface is at a predetermined height in the filter.

Here, the housing may include an upper lid disposed above the introduction region and an introduction flow path of the liquid.

Before the first step of draining a liquid, a washing solution may fill in the upstream side than the filter including the interior of the introduction region of the housing in advance.

The step of draining a liquid may include at least one step of a step of draining a fixing solution, a step of draining a permeating solution, a step of draining a staining solution, and a step of draining a washing solution; and the step of draining a cell-containing solution.

The n may be 1 and the step of draining a liquid may be the step of draining a cell-containing solution.

In the step of draining a liquid, the liquid to be drained for the first time may be a cell-containing solution, and the liquid to be drained for the second time may be a washing solution.

In the step of draining a liquid, at least part of the trapping target cell may be immersed in the liquid in the introduction region on the filter.

In the step of draining a liquid, the height of the liquid surface of the liquid in the introduction region on the filter may be equal to or greater than the size of the trapping target cell.

In the step of draining a liquid, the height of the liquid surface of the liquid in the introduction region on the filter may be 50 $\mu$m or greater.

A solvent of the cell-containing solution may include an aqueous solution containing whole blood, serum, or plasma of a mammal, or a protein originated therefrom.

The filter may be processed by using a polymeric material or a metal.

In the step of draining a liquid, a rate of introducing the liquid to the cell trapping device may be 50 to 1000 $\mu$L/min.

In addition, provided is a method for producing a specific cell-containing solution according to still another aspect of the present invention, which is obtained by selectively trapping a specific cell included in a cell-containing solution at a filter surface by filtering a liquid using a cell trapping device, the cell trapping device including a housing which has an introduction region having a predetermined volume and for introducing a liquid selected from the cell-containing solution containing two or more types of cells and a treating solution for treating the cells in the cell-containing solution into an interior, and a discharge flow path for discharging the liquid to the exterior; and a filter which is provided on a flow path between the introduction region and the discharge flow path and in which a plurality of through holes for the liquid to flow through is formed in the thickness direction, the method includes: a step of draining a liquid to the cell trapping device for n (n is a natural number) times, in which from the first step of draining the liquid to the n-th step of draining the liquid, a liquid surface of the liquid in the introduction region on the filter is maintained at a predetermined liquid surface height, and when the n-th step of draining the liquid is completed, the discharging of the liquid from the cell trapping device is stopped in a state where the liquid surface is at a predetermined height in the filter, and in which after the step of draining a liquid to the cell trapping device, a step of collecting a liquid existing in the housing is included.

The step of collecting a liquid existing in the housing may include a step of sucking a liquid in the introduction region from the introduction region side.

The housing may include an upper lid disposed above the introduction region and an introduction flow path of the liquid, and the step of collecting a liquid existing in the housing may include a step of sending a liquid from the discharge flow path to the introduction flow path.

The flow rate of sending a liquid from the discharge flow path to the introduction flow path may be 50 to 1000 $\mu$L/min.

The housing may be vibrated before a liquid is sent from the discharge flow path to the introduction flow path.

The housing may include an upper lid disposed above the introduction region and two or more of the introduction flow paths of the liquid, and the step of collecting a liquid existing in the housing may include a step of sending a liquid to an introduction flow path different from a first introduction flow path among two or more introduction flow paths from the first introduction flow path included in the two or more introduction flow paths.

A flow rate of sending a liquid to an introduction flow path different from a first introduction flow path among two or more introduction flow paths from the first introduction flow path included in the two or more introduction flow paths may be 50 to 1000 $\mu$L/min.

The housing may be vibrated before a liquid is sent to an introduction flow path different from a first introduction flow path among two or more introduction flow paths from the first introduction flow path included in the two or more introduction flow paths.

Before the first step of draining a liquid, a washing solution may fill in the upstream side than the filter including the interior of the introduction region of the housing in advance.

The step of draining a liquid may include at least one step of a step of draining a fixing solution, a step of draining a permeating solution, a step of draining a staining solution, and a step of draining a washing solution; and the step of draining a cell-containing solution.

The n may be 1 and the step of draining a liquid may be the step of draining a cell-containing solution.

In the step of draining a liquid, the liquid to be drained for the first time may be a cell-containing solution, and the liquid to be drained for the second time may be a washing solution.

In the step of draining a liquid, at least part of the trapping target cell may be immersed in the liquid in the introduction region on the filter.

In the step of draining a liquid, the height of the liquid surface of the liquid in the introduction region on the filter may be equal to or greater than the size of the trapping target cell.

In the step of draining a liquid, the height of the liquid surface of the liquid in the introduction region on the filter

may be 50 μm or greater.

**Advantageous Effects of Invention**

According to the present invention, provided is a cell trapping method, by which a specific cell is extracted without imparting damage to the trapped cell after the specific cell is trapped by filtration, a method for producing a specific cell-trapping device for extraction, and a method for producing a specific cell-containing solution containing the trapped cell.

**Brief Description of Drawings**

Fig. 1 is a view illustrating a schematic configuration of a cell trapping device (open type housing).
Fig. 2 is a view illustrating a schematic configuration of a cell trapping device (closed type housing).
Fig. 3 is a cross-sectional view schematically illustrating a method for producing a filter.
Fig. 4 is a cross-sectional view schematically illustrating a method for producing a filter using a copper plate (Ni plate in a case of performing copper plating).
Fig. 5 is a view illustrating a definition of a long pore diameter, a short pore diameter, and an aperture ratio of the filter.
Fig. 6 is a photograph of the trapped cell in Example 1.
Fig. 7 is a photograph of the trapped cell in Example 4.
Fig. 8 is a photograph of the trapped cell in Comparative Example 1.
Fig. 9 is a photograph of the trapped cell in Comparative Example 4.

**Description of Embodiments**

Hereinafter, one embodiment of the present invention will be described in detail. The cell trapping method according to the present embodiment is a method for selectively trapping a specific cell from a cell-containing solution containing two or more types of cells. When trapping the specific cell, a cell trapping device is used. The cell trapping device will be described later but the cell trapping device includes a housing which has an introduction region having a predetermined volume and a discharge flow path for introducing a liquid introduced into the interior from the introduction region to the exterior; and a filter which is provided on a flow path between the introduction region and the discharge flow path and in which a plurality of through holes for the liquid to flow through is formed in the thickness direction. By filtering the cell-containing solution using this cell trapping device, the specific cell is trapped on the filter. When filtering the cell-containing solution, it is possible to treat the trapped cell in order to perform observation by draining a treating solution such as a fixing solution, a permeating solution, a staining solution, or a washing solution to the cell trapping device. In addition, the cell trapping device in which the specific cell is trapped on the filter is referred to as a specific cell-trapping device.

In the present embodiment, the cell-containing solution is defined as a solution in which two or more types of cells exist. Examples of the cell-containing solution include human blood and a solution containing human blood.

In the present embodiment, in a case where the cell-containing solution is human blood, a cell in the cell-containing solution is a cell existing in the human blood and two or more types of cells are targets. Specific examples thereof include a human white blood cell, a circulating tumor cell (Circulating Tumor Cell: CTC), a circulating endothelial cell (Circulating Endothelial Cell: CEC), and a cancer stem cell.

A solvent of the cell-containing solution is preferably an aqueous solution containing whole blood, serum, or plasma of a mammal, or a protein originated therefrom, more preferably a protein originated from a cow, a horse, or a human, and still more preferably a protein originated from a human. The aqueous solution preferably contains a phosphate buffer solution as a major component.

An anticoagulant agent may be added to the aqueous solution containing a protein. The anticoagulant agent is preferably any one of EDTA, heparin, sodium citrate, and sodium fluoride.

The specific target cell to be trapped by the cell trapping device is a cell existing in the human blood. Specific examples thereof include a human white blood cell, a circulating tumor cell, a circulating endothelial cell, and a cancer stem cell.

In addition, in the present embodiment, a specific cell-containing solution is defined as a solution produced after the specific cell is trapped from the cell-containing solution where two or more types of cells exist using the cell trapping device. In the specific cell-containing solution, an existence ratio of the trapped specific cell to the cell other than the specific cell is higher than that of the original cell-containing solution. The specific cell-containing solution is obtained by collecting a liquid remaining in the specific cell-trapping device after the specific cell is trapped in the cell trapping device.

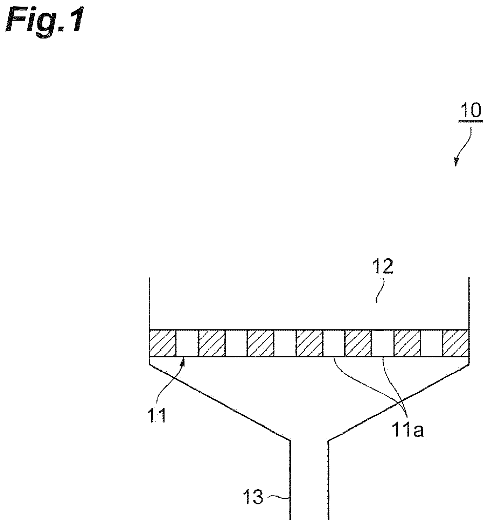

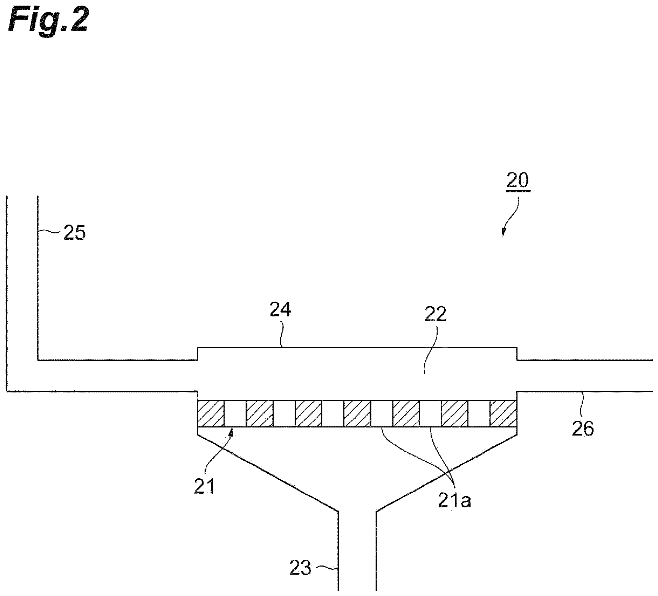

The cell trapping device will be described with reference to Fig. 1 and Fig. 2. The cell trapping device has a housing and a filter. First, the housing will be described. The housing in the cell trapping device of the present embodiment can introduce a liquid into the interior and can discharge the liquid flown through the filter. Furthermore, an introduction region having a predetermined volume is included in the housing such that a liquid surface of the liquid staying on the filter can be maintained at a predetermined height. There are two kinds of this housing. In addition, the shape of the cell trapping device is not limited to the above as illustrated in Fig. 1 or Fig. 2. A device configuration is not particularly limited as long as the specific cell can be trapped by filtering the cell-containing solution using the filter.

First, the first one is an open type housing that does not include an upper lid above than the filter. Fig. 1 illustrates a cell trapping device 10 having the open type housing. The cell trapping device 10 has a filter 11 having through holes extending in the thickness direction (vertical direction), an introduction region 12 provided on the filter 11, and a discharge flow path 13 provided below the filter. The introduction region 12 and the discharge flow path 13 are formed by the housing. In the cell trapping device 10 having the open type housing, a liquid to be introduced is introduced by the introduction region 12 existing on the filter 11. The above of the introduction region 12 is open and accordingly the liquid can be introduced directly. As a medium for introducing the liquid, a case of manually introducing the liquid by a measurer or a use of the device for automatically introducing a predetermined amount of the liquid is preferable. As the device for introducing a constant amount of the liquid, a semiautomatic device such as a burette or an automatic device which is mechanically controlled is preferable.

The second housing is a closed type housing that includes an upper lid having at least one introduction flow path for introducing a liquid above the filter and a discharge flow path for discharging the liquid to the exterior. Fig. 2 illustrates a cell trapping device 20 having the closed type housing. The cell trapping device 20 has a filter 21 having through holes extending in the thickness direction (vertical direction), an introduction region 22 provided on the filter 21, and a discharge flow path 23 provided below the filter 21. Furthermore, an upper lid 24 is provided above the introduction region 22 and two introduction flow paths 25 and 26 for introducing the liquid to the introduction region 22 covered by the upper lid 24 are provided. The introduction region 22 is formed between the upper lid 24 and the filter 21, which is a space having a predetermined volume for keeping the liquid and a cell. The introduction flow paths 25 and 26 are preferably installed above the upper lid 24 or on the transverse side of the upper lid 24. In the cell trapping device 20 of Fig. 2, an example is illustrated, in which the connection portion of the introduction flow path 25 to the introduction region is provided on the transverse side of the upper lid 24 but the flow path extends to the above. In a case where a plurality of the introduction flow paths 25 and 26 are included, it is possible to prevent liquids (for example, cell-containing solution and treating solution) to be introduced into the cell trapping device 20 from being contaminated. Accordingly, a configuration of including two or more introduction flow paths is preferable.

The filters 11 and 21 used in the cell trapping devices 10 and 20 will be described in detail, but the material is preferably a metal or a polymeric material. As a major component of the metal, any one of nickel, silver, palladium, copper, and iridium, or an alloy thereof is preferable. As a major component of the polymeric material, a material using polyethylene terephthalate, polycarbonate, polyimide, polytetrafluoroethylene, polyurethane, polylactic acid, or parylene as a major component is preferable. In addition, the "major component" means that a ratio of the component in the material configuring the filter is 50% by weight or more.

In addition, a plurality of through holes 11a and 21a for the liquid to flow through are formed in the thickness direction in the filters 11 and 21. By adjusting the shape and the size of these through holes 11a and 21a, it is possible to selectively trap the specific cell which is a trapping target.

Next, a method for trapping a cell in the cell trapping device will be described. First, a step of draining a liquid to the cell trapping device 10 or the cell trapping device 20 will be described. The step of draining a liquid in the present embodiment refers to an introduction of the target liquid from the above the filters 11 and 21 or from the dedicated introduction flow path (for example, introduction flow path 25). Thereafter, the liquid is made to flow through the filters 11 and 21, and then the liquid is discharged by discharge flow paths 13 and 23. A method for draining the liquid, suction is preferable and as a power for sucking the liquid, a peristaltic pump is preferably used.

In the treatment relating to the cell trapping according to the present embodiment, a step of draining a liquid is performed n times (n is a natural number). The number of the step of draining a liquid is varied based on the number of the step of draining a cell-containing solution and the number of a step of draining a treating solution (at least one step among a step of draining a fixing solution, a step of draining a permeating solution, a step of draining a staining solution, and a step of draining a washing solution). That is, in a case where n is 1, only the step of draining a cell-containing solution is performed. In addition, by configuring such that the liquid to be drained for the first time is set to a cell-containing solution and the liquid to be drained for the second time is set to a washing solution, the treatment using various treating solutions can be executed after the component other than the specific cell which is a trapping target among the components contained in the cell-containing solution is washed (moved to the discharge flow path side) by a washing solution. Thus, it is possible to enhance the effect of the treatment using the treating solution other than the washing solution.

When n times of the step of draining a liquid are included, in the first step of draining a liquid to the n-th step of draining a liquid, a liquid surface of the liquid staying in the introduction regions 12 and 22 on the filters 11 and 21 is maintained at a predetermined height. Adoption of this configuration enables to realize that at least part of the trapping target cell is immersed in a liquid in the introduction region on the filter. Also, after the n-th final step of draining a liquid (at the time of completion), in a state where the liquid staying in the introduction regions 12 and 22 on the filters 11 and 21 has a predetermined liquid surface height, the discharging of the liquid from the cell trapping devices 10 and 20 is stopped. The predetermined height is preferably the height equal to or greater than the average particle diameter of the trapping target cell and more preferably equal to or greater than the size of the trapping target cell. Specifically, the liquid surface height of the liquid staying in the introduction regions 12 and 22 on the filters 11 and 21 is preferably 50 $\mu$m or greater and more preferably 30 $\mu$m or greater. As the liquid height is set to the height equal to or greater than the average particle diameter of the trapping target cell, mobility of the specific cell staying on the filter can be sufficiently secured and damage of the cell caused by collision with the filter or other cells can be prevented.

Before the first step of draining a liquid, a washing solution preferably fills in the upstream side than the filter including the introduction region in advance, in other words, in the introduction region and the flow path (introduction flow path, discharge flow path) within the housing. As the washing solution, a phosphate buffer solution including a serum originated from a cow is preferably used and a phosphate buffer solution including a fetal bovine serum is more preferably used. The concentration of the bovine serum is preferably 0.1 w/v% to 20 w/v% and more preferably 0.5 w/v% to 10 w/v%.

A flow rate of the cell-containing solution and various treating solutions to flow through the filter pore is preferably in the range of 100 to 800 $\mu$L/min and more preferably in the range of 200 to 600 $\mu$L/min.

In the cell trapping device 20 having a closed type housing, in a state where the liquid is filled in the housing after trapping the cell, after the discharging of the liquid from the discharge flow path 23 is stopped, the device can be used as a container for storing the cell in a state where the shape of the trapped cell is kept.

in addition, after the cell is trapped on the filter of the cell trapping device, a method for collecting the cell-containing solution existing in the housing as a specific cell-containing solution differs depending on an open type housing and a closed type housing.

For example, a method for collecting the cell-containing solution from the cell trapping device 10 having an open type housing, a suction device such as a pipette is preferably used.

As a method for collecting the cell-containing solution from the cell trapping device (for example, a cell trapping device 20) having an closed type housing, three methods are exemplified. First, as the first method, a method is exemplified, which is used in a case where two or more introduction flow paths such as the introduction flow paths 25 and 26 are included. An introduction flow path (first introduction flow path) on one side is set as an introduction side of the liquid, an introduction flow path different from the introduction flow path used as the introduction side of the liquid is set as a discharge side, and for example, a treating solution or the like is sent as the liquid. Due to this, the cell-containing solution containing the specific cell trapped by the filter 21 is collected from the introduction flow path set as the discharge side to obtain a specific cell-containing solution. At this time, the rate of sending the liquid is preferably in the range of 50 to 1000 $\mu$L/min and more preferably in the range of 200 to 800 $\mu$L/min. As the rate of sending the liquid is set to the aforementioned range, it is possible to increase efficiency of collecting the specific cell on the filter 21 without damaging the specific cell.

As the second method, a method is exemplified which is used in a case where one introduction flow path is included. A discharge flow path is set as an introduction side of the liquid and an introduction flow path is set as a discharge side of the liquid. As the liquid is sent, the cell-containing solution is collected from the introduction flow path set as the discharge side. At this time, the rate of sending the liquid is preferably in the range of 50 to 1000 $\mu$L/min and more preferably in the range of 200 to 800 $\mu$L/min.

At the time when the cell-containing solution is collected from a closed type housing, it is preferable to vibrate the housing constantly before sending the liquid. As a medium for imparting vibration, Mixer or Shaker is preferably used.

As the third method, a suctioning method is exemplified in the same manner as the cell trapping device 10 having an open type housing. In this case, it is considered that a suction device such as a pipette is connected to one introduction flow path to perform suctioning.

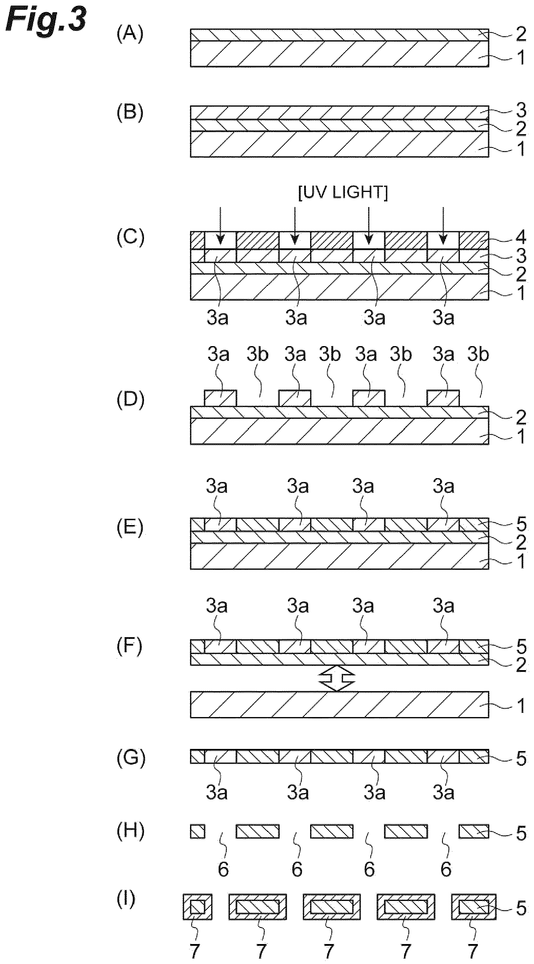

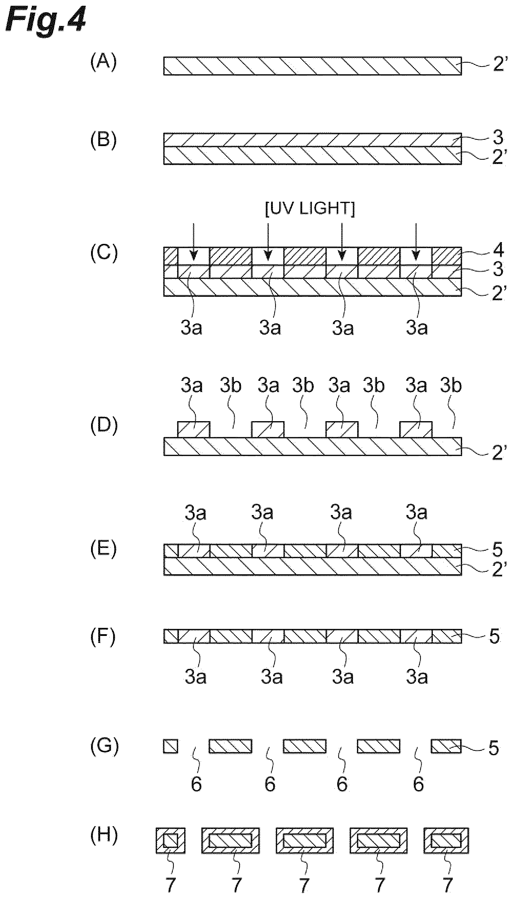

Hereinafter, a method for producing a metal filter which is one type of a filter preferably used in the cell trapping devices 10 and 20 will be exemplified with reference to Fig. 3 and Fig. 4 and the metal filter will be described.

Fig. 3 is a cross-sectional view schematically illustrating a method for producing a thin film filter made of a metal using a substrate in which a peelable copper foil (Ni foil in a case of performing copper plating) is attached to MCL.

Fig. 3(A) illustrates a substrate in which a peelable copper foil 2 (Ni foil in a case of performing copper plating) is laminated on a MCL 1. First, this substrate is prepared.

A material used as the substrate in order to produce a filter is preferably copper (nickel in a case where plating is copper). Copper can be easily removed by chemical dissolution using a chemical solution, and is excellent in adhesion to a photoresist compared with other materials.

Next, as illustrated in Fig. 3(B), a photoresist 3 is formed on the peelable copper foil 2 of the substrate. The thickness of this photoresist is preferably 1.0 time to 2.0 times the thickness of the conductor described later. If the thickness is small, resist peeling is difficult later and if the thickness is large, circuit formation is difficult. Specifically, the thickness of the photoresist 3 is preferably 15 to 50 $\mu$m. As a photosensitive resin composition forming the photoresist 3, a negative-type photosensitive resin composition is preferable. The negative-type photosensitive resin composition preferably includes at least a binder resin, a photopolymerizable compound having an unsaturated bond, and a photopolymerization initiator.

Next, as illustrated in Fig. 3(C), the photoresist is exposed after a photomask 4 is overlapped on the photoresist 3. Due to this, an exposed area 3a is formed on the area where the photomask 4 is not overlapped.

Next, as illustrated in Fig. 3(D), the photoresist of an unexposed area 3b is developed and removed by an alkaline solution or the like. Due to this, the exposed area 3 a remains in the position corresponding to the through hole of the filter.

Next, as illustrated in Fig. 3(E), a plated layer 5 is formed by performing electrolytic plating on the area which is not covered by the photoresist. This plated area is a material of the filter.

Next, as illustrated in Fig. 3(F), the peelable copper foil 2 in which the plated layer 5 is formed by electrolytic plating is peeled off from the MCL 1.

Next, as illustrated in Fig. 3(G), the peelable copper foil 2 is removed by chemical dissolution using a chemical solution and a self-supporting film formed by the exposed area 3a and the plated layer 5 is extracted.

Next, as illustrated in Fig. 3(H), the exposed area 3a of the photoresist remaining within the self-supporting film is removed to form a through hole 6 with respect to the plated layer 5.

Next, as illustrated in Fig. 3(I), electroless gold plating is performed to form a gold plated layer 7 on the filter surface. According to the above, the filters 11 and 21 can be produced which are used in the cell trapping devices 10 and 20.

Fig. 4 is a cross-sectional view schematically illustrating a method for producing a thin film filter made of a metal using a copper plate (Ni plate in a case of performing copper plating).

In the method illustrated in Fig. 4, the method is the same as the method illustrated in Fig. 2 except that a copper plate (or Ni plate) 2' is used instead of the MCL 1 and the peelable copper foil 2 in the method illustrated in Fig. 3.

That is, Fig. 4(A) illustrates a step of preparing a copper plate 2' used as the substrate. Fig. 4(B) illustrates a step of laminating the photoresist 3 on the copper plate 2'. Fig. 4(C) illustrates an exposure of the photoresist with the photomask 4 overlapped thereon. Fig. 4(D) illustrates a development and removal of the photoresist of the unexposed area 3b. Fig. 4(E) illustrates a step of forming a plated layer by electrolytic plating of the portion which is not covered by the exposed area 3a of the photoresist. Fig. 4(F) illustrates a step of removing the copper plate 2' by chemical dissolution (chemical etching) using a chemical solution and extracting a self-supporting film formed by the exposed area 3a and the plated layer 5. Fig. 4(G) illustrates a step of removing the exposed area 3a of the photoresist remaining within the self-supporting film and forming the through hole 6 with respect to the plated layer 5. Fig. 4(H) illustrates a step of performing electroless gold plating to form a gold plated layer 7 on the filter surface.

As such, the filters 11 and 21 used in the cell trapping devices 10 and 20 can be produced using any of the method illustrated in Fig. 3 and the method illustrated in Fig. 4.

The material of the filter is preferably a metal. As a major component of the metal, any one of nickel, silver, palladium, copper, and iridium or an alloy thereof is preferable. Electroplating can be performed with respect to the above metal. In addition, the "major component" means that a weight ratio of the component with respect to the total weight of the filter 105 is 50% by weight or more.

Palladium and iridium have satisfactory properties such as a high oxidation-reduction potential and poor solubility but have a disadvantage, which is expensive. Since nickel has a low oxidation-reduction potential than hydrogen, nickel is likely to be dissolved and inexpensive. Silver and palladium are precious metals and relatively inexpensive compared to iridium.

The material used as the substrate (copper plate 2': refer to Fig. 4) for producing the filter is preferably copper (in a case where the material of the filter is copper, nickel). The copper can be easily removed by chemical dissolution using a chemical solution and is excellent in adhesion to a photoresist compared with other materials. In addition, the metal foil 2 (refer to Fig. 3) laminated on the MCL 1 is preferably copper (in a case where the material of the filter is copper, nickel).

In addition, the plated layer 5 illustrated in Fig. 3(E) and Fig. 4(E) is formed by electrolytic plating. Examples of the electrolytic nickel plating include Watts bath (nickel sulfate, nickel chloride, and boric acid are major components), sulfamate bath (nickel sulfamate and boric acid are major components), and strike bath (nickel chloride and hydrogen chloride are major components).

Examples of the electrolytic silver plating include a bath using silver potassium cyanide or potassium tartrate as a major component.

Examples of the electrolytic palladium plating include a bath including water-soluble palladium salts and naphthalene sulfonic acid compound.

Examples of the electrolytic iridium plating include a bath including soluble iridium salts containing halogen and alcohols.

Examples of the electrolytic copper plating include a bath including copper sulfate, sulfuric acid, and chloride ion as a major component.

Electrolytic plating is performed using these plating baths. The current density at the time of electrolytic plating is preferably in the range of 0.3 to 4 $A/dm^2$ and more preferably in the range of 0.5 to 3 $A/dm^2$. By setting the current density to 4 $A/dm^2$ or less, generation of roughness is suppressed, and by setting the current density to 0.3 $A/dm^2$ or more, a crystal particle of the metal grows sufficiently and an effect as a barrier layer is increased. Thus, an effect of the present embodiment is obtained satisfactorily.

The resist part at the time of performing plating becomes the through hole part. Examples of the opening shape of the through hole include a circle, an oval, a square, a rectangle, a rounded rectangle, and a polygon. A circle, a rectangle, or a rounded rectangle is preferable from a viewpoint of being able to trap the target component more effectively. Also, a rounded rectangle is particularly preferable from a viewpoint of preventing clogging of the filter.

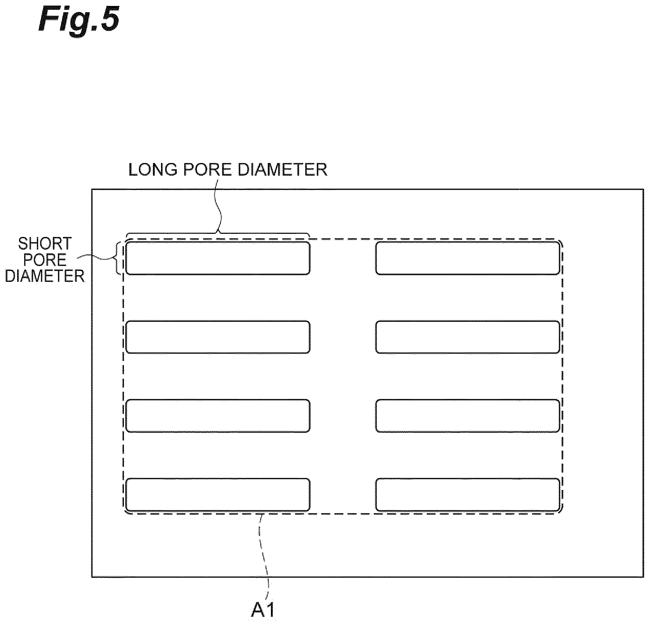

The pore diameter of the through hole 6 is set depending on the size of the trapping target component. In the present specification, the pore diameter, in a case where the opening shape is the shape other than a circle such as an oval, a rectangle, and a polygon, is set to the maximum value of the diameter of each sphere which can pass through the through hole. For example, in a case where the opening shape is a rectangle, as illustrated in Fig. 5, a short pore diameter of the short side and a long pore diameter of the long side can be defined, but the pore diameter of the through hole 6 becomes the short pore diameter. In addition, for example, in a case where the opening shape is a rectangle, the pore diameter of the through hole becomes the length of the short side of the rectangle, and in a case where the opening shape is a polygon, the pore diameter becomes the diameter of the inscribed circle of the polygon. In a case where the opening shape is a rectangle or a rounded rectangle, even in a state where the trapping target cell is trapped in the through hole 6, in the opening, a gap is generated in the long side direction of the opening shape. Since the liquid can be flow through this gap, clogging of the filter can be prevented. The length of the short pore diameter of the metal filter is preferably 5 to 15 $\mu$m and more preferably 7 to 9 $\mu$m.

The average aperture ratio of the through hole of the filters 11 and 21 is preferably 3% to 50%, more preferably 3% to 20%, and particularly preferably 3% to 10%. Here, the aperture ratio refers to a ratio of the area where the through hole occupies with respect to the area of the region functioning as the filter (refer to Fig. 5). That is, the area of the region functioning as the filter is a region (region A1 surrounded by a broken line in Fig. 5) obtained by linking the outermost part of the through hole among a plurality of through holes included in the filter. If the aperture ratio is too great, pressure applied to a white blood cell is decreased and a residue of the white blood cell is increased. If the aperture ratio is low, a blood amount to be drained is decreased.

The thickness of the filters 11 and 21 is preferably 3 $\mu$m to 50 $\mu$m, more preferably 5 $\mu$m to 30 $\mu$m, and particularly preferably 8 $\mu$m to 20 $\mu$m. In a case where the film thickness of the filters 11 and 21 is less than 3 $\mu$m the strength of the filter may be decreased and handleability may become difficult. On the contrary, if the film thickness exceeds 50 $\mu$m, it is concerned that productivity is lowered because of an increase in processing time, a disadvantageous cost is taken because of unnecessary consumption of the material, or fine processing becomes difficult, and a cell other than the trapping target specific cell such as a white blood cell is unlikely to flow through the filter.

Finally, after a circuit is formed, a resin layer is peeled off, and a copper foil is etched (Fig. 3) or a copper plate is removed (Fig. 4), and accordingly a filter using the plated layer 5 is completed as illustrated in Fig. 3(H) or Fig. 4(G).

Next, the resist (exposed area 3a) remaining in the filter is removed by strong alkali. As the strong alkali, 0.1 wt% to 10 wt% NaOH or KOH aqueous solution is preferable. Monoethanolamine (1 to 20 vol%) or the like may be added in order to promote the peeling. In a case where the peeling is difficult, the resist can be removed in a solution in which alkali (0.1 wt% to 10 wt% NaOH or KOH) is added to sodium permanganate or potassium permanganate.

A precious metal plating may be performed with respect to the filter in which the resist has been removed.

As the precious metal plating, gold, palladium, platinum, ruthenium, or indium is desirable.

Among the precious metal plating, it is considered that gold has the highest oxidation-reduction potential among all of the metals as described above and has no cytotoxicity. The color is hardly changed when stored for a long period of time.

An electroless gold plating may be performed or an electrolytic gold plating may be performed. In a case of performing electrolytic gold plating, an irregularity in the thickness becomes greater, which easily affects the accuracy of the pore diameter of the filter. Thus, it is desirable to perform electroless gold plating. However, the electrolytic gold plating can enhance a coverage factor.

The gold plating is effective only by performing a substitution plating, but a combination of the substitution plating and a reduction plating is more effective.

A metal filter before the gold plating may have an oxidized surface. Thus, removal of the oxidized film is performed, but here the filter may be washed by an aqueous solution including a metal ion and a compound forming a complex.

Specifically, aqueous solutions including cyanogens, EDTAs, or citric acids are preferable.

Among these, citric acids are optimal for pre-treatment of the gold plating. Specifically, an anhydride of citric acid, a hydrate of citric acid, citrate salts, or a hydrate of citrate salts is preferable. Specifically, citric acid anhydride, citric acid monohydrate, sodium citrate, or potassium citrate can be used. The concentration thereof is preferably 0.01 mol/L to 3 mol/L, more preferably 0.03 mol/L to 2 mol/L, and particularly preferably 0.05 mol/L to 1 mol/L. As the concentration is set to 0.01 mol/L or more, an adhesion between electroless gold plated layer and the metal filter is enhanced.

In a case where the concentration exceeds 3 mol/L, the effect is not enhanced which is not preferable in an economical aspect.

The immersion in a solution including citric acid may be performed at a temperature of 70°C to 95°C for 1 to 20 minutes.

The solution including citric acid can be added with a reducing agent included in a plating solution or a buffer agent such as a pH adjuster within the range in which the effect of the invention is obtained, but a small amount of the reducing agent or the pH adjuster is desirable and an aqueous solution only including citric acid is most preferable. The pH of the solution including citric acid is preferably 5 to 10 and more preferably 6 to 9.

The pH adjuster is not particularly limited as long as the adjuster is acid or alkali and as the acid, hydrochloric acid, sulfuric acid, or nitric acid can be used and as the alkali, a hydroxide solution of alkaline metals such as sodium hydroxide, potassium hydroxide, and sodium carbonate or alkaline earth metals is exemplified. As described above, the pH adjuster can be used within the range in which the effect of citric acid is not impaired. In addition, if nitric acid is contained at a high concentration such as 100 ml/L in the solution including citric acid, an effect of improving adhesive properties is deteriorated compared to the case of treating with the solution only including citric acid.

The reducing agent is not particularly limited as long as the agent has reducibility and examples thereof include hypophosphorous acid, formaldehyde, dimethylamine borane, and sodium borohydride.

Next, a substitution gold plating is performed. A cyanogen bath or a non-cyanogen bath can be used for the substitution gold plating, a non-cyanogen bath is desirable in consideration of an environmental load or cytotoxicity when cyanogen remains. Examples of the gold salts included in the non-cyanogen bath include chloroauric acid salts, gold sulfite salts, gold thiosulfate salts, and gold thiomalate salts. One type of the gold salts may be used alone or two or more in combination.

Furthermore, in the cyanogen-based bath, an effect of dissolving a metal is too strong and a pinhole is likely to be generated by dissolution of the metal. As described above, a non-cyanogen-based plating bath is preferable in a case where the pretreatment is performed sufficiently.

As the supply source of the gold, gold sulfite is particularly preferable. As the gold sulfite, sodium gold sulfite, potassium gold sulfite, or ammonium gold sulfite is preferable.

The gold concentration is preferably in the range of 0.1 g/L to 5 g/L. If the gold concentration is less than 0.1 g/L, gold is unlikely to be precipitated and if the gold concentration exceeds 5 g/L, the liquid is likely to be decomposed.

As a complexing agent of the gold for the substitution gold plating bath, ammonium salts or ethylene diamine tetraacetate may be added. Examples of the ammonium salts include ammonium chloride and ammonium sulfate, and as the ethylene diamine tetraacetate, ethylene diamine tetraacetic acid, ethylene diamine tetraacetic acid sodium, ethylene diamine tetraacetic acid potassium, or ethylene diamine tetraacetic acid ammonium can be used. The concentration of the ammonium salts is preferably used in the range of $7\times10^3$ mol/L to 0.4 mol/L, and if the concentration of the ammonium salts is out of this range, the liquid tends to be unstable. The concentration of the ethylene diamine tetraacetate is preferably used in the range of $2\times10^{-3}$ mol/L to 0.2 mol/L, and if the ethylene diamine tetraacetate is out of this range, the liquid tends to be unstable.

In order to maintain stability of the liquid, 0.1 g/L to 50 g/L of sulfite salts may be added. Examples of the sulfite salts include sodium sulfite, potassium sulfite, and ammonium sulfite.

In a case of decreasing the pH, as the pH adjuster, hydrochloric acid or sulfuric acid is preferably used. In addition, in a case of increasing the pH, sodium hydroxide, potassium hydroxide, or ammonia water is preferably used. The pH may be adjusted to 6 to 7. If the pH is out of this range, a bad influence is imparted to stability of the liquid or appearance of the plating.

The substitution plating is preferably performed at a liquid temperature of 30 degrees to 80 degrees, and if the liquid temperature is out of this range, a bad influence is imparted to stability of the liquid or appearance of the plating.

In this way, the substitution plating is performed, but in the substitution plating, it is difficult to cover the metal completely. Thus, next, a reduction type gold plating with a reducing agent added thereto is performed. The thickness of the substitution plating is preferably in the range of 0.02 to 0.1 $\mu$m.

As the gold salts of the reduction type gold plating, gold sulfite salts and thiosulfate salts are preferable. The gold content thereof is preferably in the range of 1 to 10 g/L. If the gold content is less than 1 g/L, a precipitation reaction of the gold is degraded and if the gold content exceeds 10 g/L, stability of the plating solution is degraded and a consumption amount of the gold is increased due to emission of the plating solution, which is not preferable. The content is more preferably 2 to 5 g/L.

Examples of the reducing agent include hypophosphorous acid, formaldehyde, dimethylamine borane, and sodium borohydride and a phenyl compound-based reducing agent is more preferable. Examples thereof include phenol, o-cresol, p-cresol, o-ethyl phenol, p-ethyl phenol, t-butyl phenol, o-aminophenol, p-aminophenol, hydroquinone, catechol, pyrogallol, methyl hydroquinone, aniline, o-phenylene diamine, p-phenylene diamine, o-toluidine, o-ethyl aniline, and p-ethyl aniline, and one type or two or more types thereof may be used.

The content of the reducing agent is preferably 0.5 g/L to 50 g/L. If the content of the reducing agent is less than 0.5 g/L, there is a tendency that it is difficult to obtain a practical precipitation rate. If the content of the reducing agent exceeds 50 g/L, stability of the plating solution tends to be degraded. The content of the reducing agent is more preferably 2 g/L to 10 g/L and particularly preferably 2 g/L to 5 g/L.

An electroless gold plating solution may contain heavy metal salts. From a viewpoint of promoting the precipitation rate, the heavy metal salts are preferably at least one selected from the group consisting of thallium salts, lead salts, arsenic salts, antimony salts, tellurium salts, and bismuth salts.

Examples of the thallium salts include inorganic compound salts such as thallium sulfate salts, thallium chloride salts, thallium oxide salts, and thallium nitrate salts; and organic complex salts such as dithallium malonate salts. Examples of the lead salts include inorganic compound salts such as lead sulfate salts and lead nitrate salts; and organic acetate salts such as acetate salts.

In addition, examples of the arsenic salts include inorganic compound salts such as arsenous acid salts, arsenic acid salts, and arsenic trioxide; and organic complex salts. Examples of the antimony salts include organic complex salts such as antimony tartrate salts; antimony chloride salts; and inorganic compound salts such as antimony oxysulfate salts and antimony trioxide.

Examples of the tellurium salts include inorganic compound salts such as tellurite salts and tellurate salts; and organic complex salts. Examples of the bismuth salts include inorganic compound salts such as bismuth sulfate (III), bismuth chloride (III), bismuth nitrate (III); and organic complex salts such as bismuth oxalate (III).

One or more types of the aforementioned heavy metal salts may be used, but the total addition amount thereof is preferably 1 to 100 ppm and more preferably 1 to 10 ppm based on the total volume of the plating solution. If the total addition amount is less than 1 ppm, the effect of enhancing the precipitation rate may not be sufficient and if the total addition exceeds 100 ppm, stability of the plating solution tends to be degraded.

The electroless gold plating solution may contain a sulfur-based compound. As the sulfur compound is further contained in the phenyl compound-based reducing agent and the electroless gold plating solution containing the heavy metal salts, even if the liquid temperature is low such as about 60°C to 80°C, a sufficient precipitation rate is obtained, the appearance of a coating film is satisfactory, and stability of the plating solution is particularly excellent.

Examples of the sulfur-based compound include sulfide salts, thiocyanate salts, thiourea compound, mercaptan compound, sulfide compound, disulfide compound, thioketone compound, thiazole compound, and thiophene compound.

Examples of the sulfide salts include potassium sulfide, sodium sulfide, sodium polysulfide, and potassium polysulfide. Examples of the thiocyanate salts include sodium thiocyanate, potassium thiocyanate, and potassium dithiocyanate. Examples of the thiourea compound include thiourea, methyl thiourea, and dimethyl thiourea.

Examples of the mercaptan compound include 1,1-dimethyl ethane thiol, 1-methyl-octane thiol, dodecane thiol, 1,2-ethane dithiol, thiophenol, o-thiocresol, p-thiocresol, o-dimercaptobenzene, m-dimercaptobenzene, p-dimercaptobenzene, thioglycol, thiodiglycol, thioglycolic acid, dithioglycolic acid, thiomalic acid, mercaptopropionic acid, 2-mercaptobenzimidazole, 2-mercapto 1-methyl imidazole, and 2-mercapto-5-methyl benzimidazole.

Examples of the sulfide compound include diethyl sulfide, diisopropyl sulfide, ethyl isopropyl sulfide, diphenyl sulfide, methyl phenyl sulfide, rhodanine, thiodiglycolic acid, and thiodipropionic acid. Examples of the disulfide compound include dimethyl disulfide, diethyl disulfide, and dipropyl disulfide.

Examples of the thioketone compound include thiosemicarbazide. Examples of the thiazole compound include thiazole, benzothiazole, 2-mercaptobenzothiazole, 6-ethoxy-2-mercaptobenzothiazole, 2-aminothiazole, 2,1,3-benzothiadiazole, 1,2,3-benzothiadiazole, (2-benzothiazolyl thio)acetic acid, and 3-(2-benzothiazolyl thio)propionic acid. Examples of the thiophene compound include thiophene and benzothiophene.

The sulfur-based compound may be used alone or two or more types thereof may be used. The content of the sulfur-based compound is preferably 1 ppm to 500 ppm, more preferably 1 ppm to 30 ppm, and particularly preferably 1 ppm to 10 ppm. If the content of the sulfur-based compound is less than 1 ppm, the precipitation rate is decreased, a failure of the covering power in plating is generated, and the appearance of a coating film is deteriorated. If the content of the sulfur-based compound exceeds 500 ppm, it is difficult to manage the concentration and the plating solution becomes unstable.

In addition to the aforementioned gold salts, the reducing agent, the heavy metal salts and the sulfur-based compound, the electroless gold plating solution preferably contains at least one of a complexing agent, a pH buffer agent, and a metal ion masking agent and more preferably contains all of them.

The electroless gold plating solution of the present embodiment preferably contains a complexing agent. Specific examples thereof include a non-cyanogen-based complexing agent such as sulfite salts, thiosulfate salts, and thiomanate salts. The content of the complexing agent is preferably 1 g/L to 200 g/L based on the total volume of the plating solution. In a case where the content of the complexing agent is less than 1 g/L, a gold complexing power is decreased and stability is decreased. In a case where the content of the complexing agent exceeds 200 g/L, plating stability is enhanced but recrystallization occurs in the liquid, which is not favorable in an economical aspect. The content of the complexing agent is preferably set to 20 g/L to 50 g/L.

The electroless gold plating solution preferably contains a pH buffer agent. The pH buffer agent has an effect of maintaining the precipitation rate to a constant value and stabilizing the plating solution. A plurality of substances may be mixed for the buffer agent. Examples of the pH buffer agent include phosphate salts, acetate salts, carbonate salts, borate salts, citrate salts, and sulfate salts. Among these, borate salts and sulfate salts are particularly preferable.

The content of the pH buffer agent is preferably 1 g/L to 100 g/L based on the total volume of the plating solution. If the content of the pH buffer agent is less than 1 g/L, a pH buffering effect is not exhibited and if the content of the pH buffer agent exceeds 100 g/L, recrystallization may occur. More preferable content is 20 g/L to 50 g/L.

The gold plating solution preferably contains a masking agent. As the masking agent, a benzotriazole-based compound can be used and examples of the benzotriazole-based compound include benzotriazole sodium, benzotriazole potassium, tetrahydrobenzotriazole, methyl benzotriazole, and nitrobenzotriazole.

The content of the metal ion masking agent is preferably 0.5 g/L to 100 g/L based on the total volume of the plating solution. If the content of the metal ion masking agent is less than 0.5 g/L, there is a tendency that an impurity masking effect is small and sufficient liquid stability cannot be secured. Meanwhile, if the content of the metal ion masking agent exceeds 100 g/L, recrystallization may occur in the plating solution. In view of the cost and the effect, the range of 2 g/L to 10 g/L is most preferable.

The pH of the gold plating solution is preferably in the range of 5 to 10. In a case where the pH of the plating solution is less than 5, sulfite salts or thiosulfate salts, which are a complexing agent of the plating solution, may be decomposed and a toxic sulfurous acid gas may be generated. In a case where the pH of the plating solution exceeds 10, stability of the plating solution tends to be decreased. In order to enhance the precipitation efficiency of the reducing agent and obtain the fast precipitation rate, the pH of the electroless gold plating solution is preferably in the range of 8 to 10.

As the electroless plating method, a filter in which the substitution gold plating has been completed is immersed to perform the gold plating.

The plating temperature may be 50°C to 95°C. If the plating temperature is lower than 50°C, the precipitation efficiency is deteriorated and if the plating temperature is 95°C or higher, the liquid tends to be unstable.

The gold plated layer 7 which is the outermost layer and formed in this way preferably includes gold having purity of 99% by weight or more. If the purity of the gold of the gold plated layer 7 is less than 99% by weight, cytotoxicity of a contact portion becomes higher. From a viewpoint of increasing reliability, the purity of the gold of the gold plated layer 7 is more preferably 99.5% by weight.

In addition, the thickness of the gold plated layer 7 is preferably 0.005 to 3 $\mu$m, more preferably 0.05 to 1 $\mu$m, and still more preferably 0.1 $\mu$m to 0.5 $\mu$m. As the thickness of the gold plated layer 7 is set to 0.005 $\mu$m or more, exudation of the gold plated layer 7 can be suppressed at a certain degree. Meanwhile, even if the thickness of the gold plated layer exceeds 3 $\mu$m, the effect is not greatly enhanced more than the above, and the thickness is preferably set to 3 $\mu$m or less from a viewpoint of an economical aspect.

In addition, a filter configured by a polymeric material can be used instead of the metal filter. In a case where the polymeric material is used as a material for the filter, since the molding workability is excellent compared to the metal, for example, it is possible to control the shape of the through hole or the pore diameter more finely. Also, in a case where polyethylene terephthalate, polycarbonate, polyimide, polytetrafluoroethylene, polyurethane, polylactic acid, parylene, or the like is adopted as a major component of the filter configured by the polymeric material, these materials have strong antithrombotic properties.

A method for producing a filter configured by the polymeric material is not particularly limited and for example, a dry etching method can be used. Specifically, a polymeric film having a thickness corresponding to the thickness of the filter is prepared, the polymeric film is irradiated with ion beams, neutral particle ray beams, or laser beams to form a through hole. If necessary, after irradiation, a through hole may be formed by chemical etching. By cutting the polymeric film with the through hole formed thereon according to the shape of the filter, it is possible to obtain a filter configured by the polymeric material.

In this way, the filter used in the cell trapping device according to the present embodiment can be appropriately modified.

As above, the embodiment of the present invention is described, but the cell trapping method, the method for producing a specific cell-trapping device, and a method for producing a specific cell-containing solution according to the present invention can be appropriately modified.

For example, in the cell trapping method according to one aspect of the present invention, in the step of draining a liquid, the height of the liquid surface on the filter can be 30 $\mu$m or more.

In addition, whole blood, serum, or plasma of a mammal can be originated from any one of a cow, a horse, and a human.

Whole blood, serum, or plasma of a mammal can be originated from a human.

An aqueous solution containing a protein can include a phosphate buffer solution as a major component.

An aqueous solution containing a protein can include an anticoagulant agent.

The anticoagulant agent can be any one of EDTA, heparin, sodium citrate, and sodium fluoride.

Cells in the cell-containing solution can be cells existing in the human blood.

Cells in the cell-containing solution can include any one of a human white blood cell, a circulating tumor cell, a circulating endothelial cell, and a cancer stem cell.

The filter can be molded using polyethylene terephthalate, polycarbonate, or parylene.

The filter surface can be gold, platinum, palladium, or an alloy thereof.

The filter can include nickel, copper, or palladium as a major component and the surface thereof can be plated with gold, platinum, palladium, or an alloy thereof.

The outermost layer of the filter can be gold.

The thickness of the outermost layer of the filter can be 0.05 $\mu$m to 1 $\mu$m of the precious metal plating.

The shape forming the through hole of the filter can be any one of a circle, an oval, a rounded rectangle, a rectangle, and a square.

The shape forming the through hole of the filter can include at least one of the rectangle and the rounded rectangle shape, and the length of the short side can be 5 $\mu$m to 15 $\mu$m.

The film thickness of the filter can be 3 $\mu$m to 50 $\mu$m.

The average aperture ratio of the through hole can be 0.1 % to 50%.

In addition, in the method for producing a specific cell-trapping device according to one aspect of the present invention, in the step of draining a liquid, the height of the liquid surface on the filter can be 30 $\mu$m or more.

In addition, whole blood, serum, or plasma of a mammal can be originated from any one of a cow, a horse, and a human.

Whole blood, serum, or plasma of a mammal can be originated from a human.

An aqueous solution containing a protein can include a phosphate buffer solution as a major component.

An aqueous solution containing a protein can include an anticoagulant agent.

The anticoagulant agent can be any one of EDTA, heparin, sodium citrate, and sodium fluoride.

Cells in the cell-containing solution can be cells existing in the human blood.

Cells in the cell-containing solution can include any one of a human white blood cell, a circulating tumor cell, a circulating endothelial cell, and a cancer stem cell.

The filter can be molded using polyethylene terephthalate, polycarbonate, or parylene.

The filter surface can be gold, platinum, palladium, or an alloy thereof.

The filter can include nickel, copper, or palladium as a major component and the surface thereof can be plated with gold, platinum, palladium, or an alloy thereof.

The outermost layer of the filter can be gold plated.

The thickness of the outermost layer of the filter can be 0.05 $\mu$m to 1 $\mu$m of the precious metal plating.

The shape forming the through hole of the filter can be any one of a circle, an oval, a rounded rectangle, a rectangle, and a square.

The shape forming the through hole of the filter can include at least one of the rectangle and the rounded rectangle shape, and the length of the short side can be 5 $\mu$m to 15 $\mu$m.

The film thickness of the filter can be 3 $\mu$m to 50 $\mu$m.

The average aperture ratio of the through hole can be 0.1% to 50%.

In addition, in the method for producing a specific cell-containing solution according to one aspect of the present invention, in the step of draining a liquid, the height of the liquid surface on the filter can be 30 $\mu$m or more.

Here, the solvent of the cell-containing solution can include an aqueous solution including whole blood, serum, or plasma of a mammal, or a protein originated therefrom.

Whole blood, serum, or plasma of a mammal can be originated from any one of a cow, a horse, and a human.

Whole blood, serum, or plasma of a mammal can be originated from a human.

An aqueous solution containing a protein can include a phosphate buffer solution as a major component.

An aqueous solution containing a protein can include an anticoagulant agent.

The anticoagulant agent can be any one of EDTA, heparin, sodium citrate, and sodium fluoride.

Cells in the cell-containing solution can be cells existing in the human blood.

Cells in the cell-containing solution can include any one of a human white blood cell, a circulating tumor cell, a circulating endothelial cell, and a cancer stem cell.

The filter can be molded using a polymer or a metal.

The filter can be molded using polyethylene terephthalate, polycarbonate, or parylene.

The filter surface can be gold, platinum, palladium, or an alloy thereof.

The filter can include nickel, copper, or palladium as a major component and the surface thereof can be plated with gold, platinum, palladium, or an alloy thereof.

The outermost layer of the filter can be gold plated.

The thickness of the outermost layer of the filter can be 0.05 $\mu$m to 1 $\mu$m of the precious metal plating.

The shape forming the through hole of the filter can be any one of a circle, an oval, a rounded rectangle, a rectangle, and a square.

The shape forming the through hole of the filter can include at least one of the rectangle and the rounded rectangle shape, and the length of the short side can be 5 $\mu$m to 15 $\mu$m.

The film thickness of the filter can be 3 $\mu$m to 50 $\mu$m.

The average aperture ratio of the through hole can be 0.1% to 50%.

The rate of introducing the cell solution and the treating solution can be 50 $\mu$L/min to 1000 $\mu$L/min.

[Examples]

(Evaluation Example 1)

A photosensitive resin composition (PHOTEC RD-1225: thickness of 25 $\mu$m, manufactured by Hitachi Chemical Company, Ltd.) was laminated on a single side of a 250 mm square substrate (MCL-E679F: a substrate with a peelable copper foil attached on the surface of the MCL, manufactured by Hitachi Chemical Company, Ltd.). The lamination condition was that a roll temperature is 90°C, pressure is 0.3 MPa, and a conveyor speed is 2.0 m/min.

Next, a glass mask was designed such that the shape of the portion where beams are transmitted is a rounded rectangle, the size is 7.8 (short pore diameter)×100 (long pore diameter) $\mu$m, and the aperture ratio is 6.7%. The glass mask was left standstill on a photoresist lamination surface of the substrate. In the present Examples, a glass mask, in which rounded rectangles facing the same direction are aligned at a constant pitch in the long axis and short axis direction, was used.

Subsequently, under the vacuum of 600 mmHg or less, the substrate where the glass mask is placed was irradiated with ultraviolet rays in the exposure amount of 30 mJ/cm$^2$ by an ultraviolet irradiation device from above.

Next, a development was performed using 1.0% of a sodium carbonate aqueous solution to form a resist layer with a rectangular photoresist standing upright on the substrate. The copper exposed area of the substrate with the resist was plated in a nickel plating solution, whose pH is adjusted to 4.5, at a temperature of 55°C for about 20 minutes to have a thickness of 20 $\mu$m. A composition of the nickel plating solution is shown in Table 1.

[Table 1]

| Composition of plating solution | Concentration (g/L) |
|---|---|
| Nickel sulfamate | 450 |
| Nickel chloride | 5 |
| Boric acid | 30 |

Next, the obtained nickel plated layer was peeled off with a peelable copper foil of the substrate, and the peelable copper foil was removed by chemical dissolution (MECBRITE SF-5420B, MEC Co., Ltd.) using a chemical solution under a stirring treatment at a temperature of 40°C for about 120 minutes, thereby extracting a self-supporting film (20 mm×20 mm) which is a metal filter.

Finally, the photoresist remaining in the self-supporting film was removed at a temperature of 60°C for about 40 minutes by resist peeling under an ultrasonic wave treatment (P3 Poleve, Henkel) to produce a metal filter having fine through holes.

Due to this, a metal filter having sufficiently precise through holes was produced without any damages such as folds, breakage, scratches, and curls.

Next, the metal filter was immersed in an acidic degreasing solution Z-200 (manufactured by WORLD METAL Co., Ltd.: trade name) to remove organic products on the metal filter (40°C for 3 minutes).

After the metal filter was washed with water, a pretreatment of the substitution gold plating was performed at a temperature of 80°C for 10 minutes using a solution in which gold sulfite, which is a gold supply source, has been removed from HGS-100 (manufactured by Hitachi Chemical Company, Ltd.: trade name) which is non-cyanogen-based electroless Au plating.

Next, the metal filter was immersed at a temperature of 80°C for 20 minutes in the HGS-100 (trade name manufactured by Hitachi Chemical Company, Ltd.) which is non-cyanogen-based substitution type electroless Au plating to perform the substitution gold plating. The thickness of the substitution gold plating was 0.05 $\mu$m.

After washing with water, the metal filter was immersed at a temperature of 65°C for 10 minutes in HGS-5400 (trade name manufactured by Hitachi Chemical Company, Ltd.) which is non-cyanogen-based reduction type electrodes Au plating to perform gold plating, and dried after washing with water. The total thickness of the gold plating was 0.2 $\mu$m. According to the above, a filter according to Evaluation Example 1 was produced.

(Evaluation Example 2)

A filter according to Evaluation Example 2 was produced in the same manner as Evaluation Example 1 except that electro palladium plating was used instead of electro Ni plating. PALLADEX LF-5 (Electroplating Engineers of Japan Ltd.: trade name) was used as an electro palladium plating solution. The plating was performed under a condition of the plating temperature of 50°C and the current density of 1 A/dm2, and the plating was performed under the same condition as Evaluation Example 1 except that the electroplating was performed under a condition of about 4.2 min/$\mu$m so as to be about 20 $\mu$m.

(Evaluation Example 3)

A peelable nickel foil was used instead of the peelable copper foil of the MCL (the nickel foil was removed after electroplating). Furthermore, a filter according to Evaluation Example 3 was produced on the same surface as Evaluation Example 1 except that electro copper plating was used instead of the electro Ni plating. MICROFAB Cu200 (Electroplating Engineers of Japan Ltd.: trade name) was used as an electro copper plating solution. The plating was performed under a condition of the plating temperature of 25°C and the current density of 3 A/dm2, and the plating was performed under the same condition as Evaluation Example 1 except that the electroplating was performed under a condition of about 1.5 min/$\mu$m so as to be about 20 $\mu$m.

(Evaluation Example 4)

A filter according to Evaluation Example 4 was produced in the same manner as Evaluation Example 1 except that the gold plating was not performed.

(Evaluation Example 5)

A filter according to Evaluation Example 5 was produced in the same manner as Evaluation Example 1 except that 1-stage electroless palladium plating was performed instead of 2-stage gold plating. PALLADEX STRIKE 3 (manufactured by Electroplating Engineers of Japan Ltd.: trade name) was used as an electroless palladium plating solution. The thickness of palladium was 0.1 $\mu$m.

(Evaluation Example 6)

A filter according to Evaluation Example 6 was produced in the same manner as Evaluation Example 1 except that 1-stage electroless platinum plating was performed instead of 2-stage gold plating. LECTROLESS Pt100 (manufactured by Electroplating Engineers of Japan Ltd.: trade name) was used as an electroless platinum plating solution. The thickness of palladium was 0.1 $\mu$m.

(Tests)

(Preparation of Non-small Cell Cancer Cell Strain)

NCI-H358 cells which is a non-small cell lung cancer cell strain were left standstill and cultured in a RPMI-1640 medium including 10% fetal bovine serum (FBS) under a condition of 37°C and 5% $CO_2$. The cells were peeled off from a culture dish by a trypsin treatment to collect and washed using a phosphate buffer solution (Phosphate buffered saline, PBS) and then left standstill at a temperature of 37°C for 3 minutes in a 10 $\mu$M CellTracker Red CMTPX (Life Technologies Corporation ), thereby staining the NCI-H358 cell. Thereafter, the cells were washed using the PBS and left standstill at a temperature of 37°C for 30 minutes by a trypsin treatment, thereby dissociating a cell aggregation. Thereafter, the trypsin treatment was stopped in the medium and the cells were washed using the PBS, and then suspended using the PBS including 2mMEDTA and 0.5% bovine serum albumin (BSA) (hereinafter, referred to as 2 mM EDTA-0.5% BSA-PBS), thereby obtaining a suspension for adjustment. In addition, the PBS is a phosphate buffered physiological saline and a product code 166-23555 manufactured by Wako Pure Chemical Industries, Ltd. was used. A BSA manufactured by SIGMA-ALDRICH (Product Name: Albumin from bovine serum-Lyophilized powder, Bio Reagent for cell culture) was used. 2Na (ethylenediamine-N,N,N',N'-tetraacetic acid disodium salt dihydrate) (Wako Pure Chemical Industries, Ltd. product code 345-01865) was used as the EDTA.

<Evaluation of cell state in a case where treatment is continuously performed without taking out liquid>

(Example 1)

A test was performed using a CTC collecting device: CT6000 (trade name manufactured by Hitachi Chemical Company, Ltd.) in which the filter of Evaluation Example 1 is set to a cartridge (corresponding to the cell trapping device according to the present embodiment). The CTC collecting device includes a flow path for introducing a blood sample or a reagent, and an inlet of the flow path is connected to a reservoir produced by processing a syringe. An operation such as trapping, staining, and washing of the CTC can be continuously and easily performed by sequentially putting the blood sample or the reagent into the reservoir.

The blood sample was introduced into the CTC collecting device to concentrate a cancer cell. A sample in which 1000 cancer cells per 1 mL of the blood are contained in the blood of a healthy person collected by an EDTA-containing vacuum blood collection tube was used as the blood sample. The aforementioned human non-small cell lung cancer cell NCI-H358 was used as the cancer cell. In addition, the blood was used after 6 hours from blood collection.

First, 1 ml of 2 mM EDTA-0.5% BSA (bovine serum albumin)-PBS (PBS manufactured by Gibco, pH 7.4) (hereinafter, washing solution) was introduced into the reservoir and filled on the filter and the filter was left standstill for 10 minutes. Subsequently, the liquid started to be sent at the flow rate of 200 $\mu$L/min using a peristaltic pump. Thereafter, 3 ml of the blood was put therein. After about 5 minutes, 9 mL of the washing solution was introduced into the reservoir to perform washing of the cells.

Next, a liquid in which 4% PFA is dissolved in the washing solution was put into the cartridge to immerse the cells for 15 minutes. After washing, a liquid in which 0.2% TritonX is dissolved in the washing solution was put into the cartridge to immerse the cells for 15 minutes.

Furthermore, after 10 minutes, the pump flow rate was changed to 20 $\mu$L/min and 600 $\mu$L of a cell staining solution (Hoechst 33342: 30 $\mu$L, Wash buffer: 300 ml) was introduced into the reservoir to fluorescent stain the cancer cells or the white blood cells on the filter. After the cells trapped on the filter were stained for 30 minutes, 1 mL of the 2mM EDTA-0.5% BSA-PBS was introduced into the reservoir to wash the cells.

Subsequently, the cancer cells and the white blood cells on the filter were photographed using a fluorescence microscope (BX61, Olympus Corporation) including a computer controlled type electric stage and a cooled digital camera (DP70, Olympus Corporation). In order to observe the fluorescent light originated from Hoechst 33342 and CellTracker Red CMTPX, an image was obtained using each of the WU and WIG filter (Olympus Corporation). Lumina Vision (MITANI Corporation) was used for image obtainment and analysis soft. The captured image of the cells was analyzed and the cells were classified into two, which are tetragonal cells pinched within a pore and cells kept as to have a circular shape on the filter.

(Example 2)

The test was performed under the same condition as Example 1 except that the rate of introducing the blood was 400 $\mu$L/min.

(Example 3)

The test was performed under the same condition as Example 1 except that the rate of introducing the blood was 600 $\mu$L/min.

(Example 4)

The test was performed under the same condition as Example 1 except that the rate of introducing the blood was 800 $\mu$L/min.

(Comparative Example 1)

First, 1 ml of a washing solution (hereinafter, washing solution) was introduced into a reservoir and filled on the filter and the filter was left standstill for 10 minutes. Subsequently, the liquid started to be sent at the flow rate of 200

μL/min using a peristaltic pump. Thereafter, 3 ml of the blood was put therein. After about 5 minutes, 9 mL of the washing solution was introduced into the reservoir to perform washing of the cells. Thereafter, the test was performed according to the same method as Example 1 and the cells were stained, and then the liquid within the cell trapping device (cartridge) was taken out at the rate of 200 μL/min to perform photographing using a fluorescence microscope. The captured image of the cells was analyzed and the cells were classified into two, which are cells pinched within a pore and cells kept as to have a circular shape.

(Comparative Example 2)

The test was performed according to the same method as Comparative Example 1 and the liquid within the cell trapping device was taken out at the rate of 50 μL/min to perform photographing using a fluorescence microscope.

(Comparative Example 3)

The test was performed according to the same method as Comparative Example 1 and the liquid within the cell trapping device was taken out at the rate of 2 μL/min to perform photographing using a fluorescence microscope.

<Evaluation of cell state in a case where liquid is introduced again after liquid is completely taken out once during first step of draining liquid to n-th step of draining liquid>

(Comparative Example 4)

The test was performed according to the same method as Example 1, after the staining solution was introduced, the washing was completed, and then the liquid within the cell trapping device was completely taken out at the rate of 50 μL/min. After the liquid was completely taken out, the washing solution was introduced again from the reservoir at the rate of 200 μ/min. Thereafter, the photographing was performed using a fluorescence microscope, and the captured image of the cells was analyzed and the cells were classified into two, which are cells pinched within a pore and cells kept as to have a circular shape.

(Results)

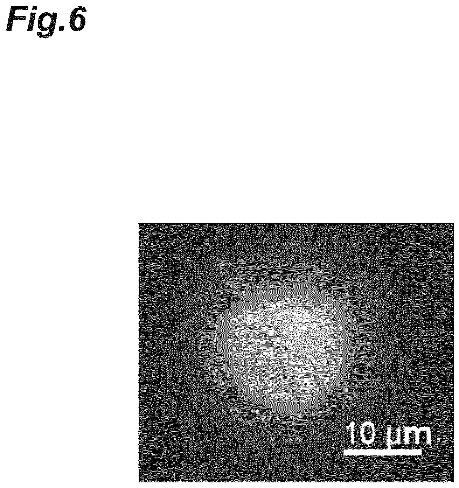

First, Fig. 6 illustrates a photograph of the trapped cells of Example 1. In Example 1, it was confirmed that the shape of the cell is circular and the particle diameter of the cell was about 18 μm which is a theoretical average particle diameter. According to this, it was suggested that by storing the cells without taking the liquid out completely after the cell trapping, it is possible to maintain the cell state and extract the cells without being pinched within a pore.

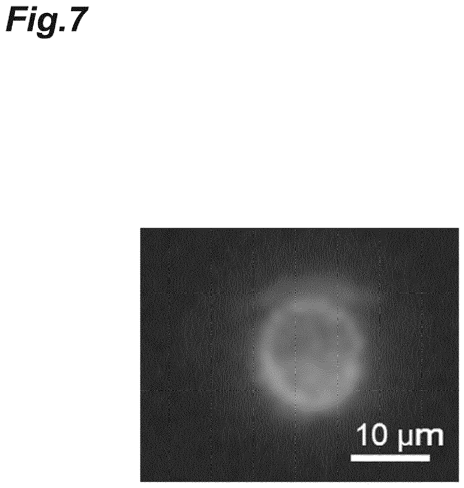

Fig. 7 illustrates a photograph of the trapped cells of Example 4. In the same manner as Example 1, it was confirmed that the shape of the cell is circular and the particle diameter of the cell was about 18 μm which is a theoretical particle diameter.

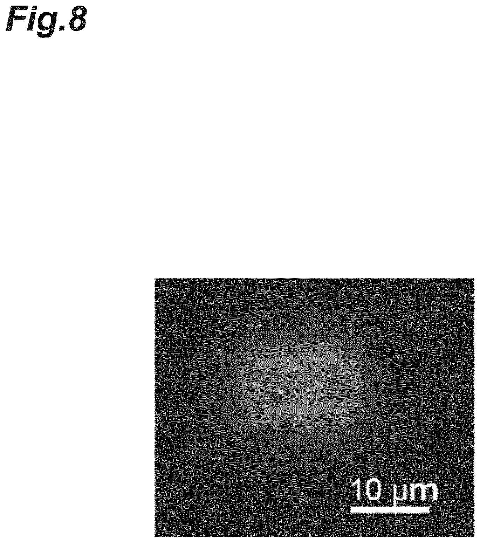

Fig. 8 illustrates a photograph of the trapped cells of Comparative Example 1. In Comparative Example 1, after the liquid was taken out, the cells trapped on the filter get caught in the through holes of the filter by the pressure of sucking the liquid, the cells are deformed to be tetragonal, and it was confirmed that the cells are pinched within a pore.

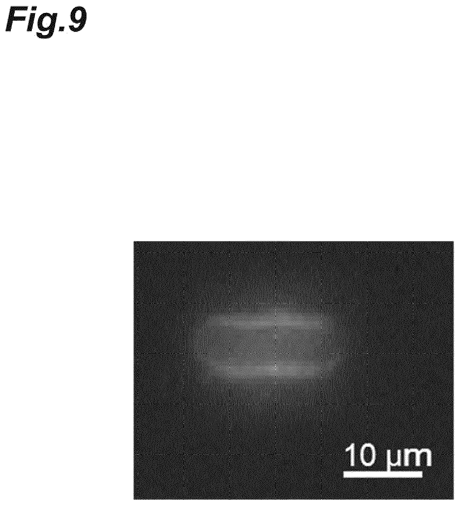

Fig. 9 illustrates a photograph of the trapped cells of Comparative Example 4. In the same manner as Comparative Example 1, the cells are deformed to be tetragonal and it was confirmed that the cells are pinched within a pore.
Next, Table 2 shows results obtained by classifying the cells in each Examples 1 to 6 and Comparative Examples 1 to 4 into two, which are cells pinched within a pore and cells kept as to have a circular shape.

[Table 2]

| | Rate of introducing blood [μL/min] | Rate of taking out liquid [μL/min] | Circular cell [%] | Tetragonal cell (pinched) [%] |
|---|---|---|---|---|
| Example 1 | 200 | - | 97.2 | 2.8 |
| Example 2 | 400 | - | 98.0 | 2.0 |
| Example 3 | 600 | - | 98.5 | 1.5 |
| Example 4 | 800 | - | 96.5 | 3.5 |
| Example 5 | 400 | - | 98.8 | 1.2 |

(continued)

| | Rate of introducing blood [μL/min] | Rate of taking out liquid [μL/min] | Circular cell [%] | Tetragonal cell (pinched) [%] |
|---|---|---|---|---|
| Example 6 | 600 | - | 97.6 | 2.4 |
| Comparative Example 1 | 400 | 200 | 2.0 | 98.0 |
| Comparative Example 2 | 400 | 50 | 0 | 100 |
| Comparative Example 3 | 400 | 2 | 1.2 | 98.8 |
| Comparative Example 4 | 400 | 50 *Thereafter, reintroduction of liquid | 0 | 100 |

As shown in Table 2, in Examples 1 to 6 in which the liquid was not taken out, 95% or more of the cells were circular and tetragonal cells were merely 5% or less. On the contrary, in Comparative Examples 1 and 2 in which the liquid was taken out, 98% or more of the cells were pinched within a pore and tetragonal. Also, in Comparative Example 3, even in a case where the liquid was taken out at low rate, which is 2 μL/min, 80% of the cells were tetragonal. Furthermore, in Comparative Example 4, even in a case where the liquid was taken out and then the liquid was introduced again, all of the cells were tetragonal.

In a case where the evaluation target specific cell (for example, CTC) is pinched within a pore, it is difficult to determine morphologically at the time of detecting the specific cell, which may lead to a misdiagnosis. In addition, since the cell is pinched within a pore, it is difficult to extract the specific cell on the filter and it is difficult to perform analysis and diagnosis of the cell. According to this, it was suggested that it is possible to stably keep the shape of the cell by continuously performing the treatment without taking out the liquid within the cell trapping device at all to the final step.

<Evaluation of cell state in a case where height of liquid surface of liquid on filter is changed>

(Example 7)

Operations such as trapping, staining, and washing of the cells were performed using the same method as Example 1. The height of the liquid from the filter surface was set to 50 μm and the liquid substitution operation was performed. The height of the liquid surface from the filter surface was calculated by a filter surface area and mass and the liquid surface was set to 50 μm.

The height of the liquid surface was calculated using the following equation.

$$\text{Height of liquid surface (μm)} = \text{Volume (μm}^3\text{)} \div \text{Surface area (μm}^2\text{)}$$

$$\text{Volume (μm}^3\text{)} = \text{Mass (μg)} \div \text{Specific gravity}$$

The image of the cells captured by using a microscope according to the same operation as Example 1 was analyzed and the cells were classified into two, which are tetragonal cells pinched within a pore and circular cells kept as to have a circular shape on the filter.

(Example 8)

The height of the liquid from the filter surface was set to 30 μm and the liquid substitution operation was performed using the same method as Example 7. The height of the liquid surface from the filter surface was calculated a filter surface area and mass and the liquid surface was set to 30 μm.

(Example 9)

The height of the liquid from the filter surface was set to 20 $\mu$m and the liquid substitution operation was performed using the same method as Example 7. The height of the liquid surface from the filter surface was calculated a filter surface area and mass and the liquid surface was set to 20 $\mu$m.

(Example 10)

The height of the liquid from the filter surface was set to 10 $\mu$m and the liquid substitution operation was performed using the same method as Example 7. The height of the liquid surface from the filter surface was calculated a filter surface area and mass and the liquid surface was set to 10 $\mu$m.

(Comparative Example 5)

The height of the liquid from the filter surface was set to 0 $\mu$m and the liquid substitution operation was performed using the same method as Example 7. At the time of exchanging the liquid, the entire liquids were taken out from the cell trapping device and then the treatment was performed and accordingly, the height of the liquid surface was set to 0 $\mu$m.

(Results)

Table 3 shows results of Example 7 to Example 10 and Comparative Example 5.

[Table 3]

|  | Height of liquid surface [$\mu$m] | Circular cell [%] | Tetragonal cell (pinched) [%] |
|---|---|---|---|
| Example 7 | 50 | 98.2 | 1.8 |
| Example 8 | 30 | 62.5 | 37.5 |
| Example 9 | 20 | 10.3 | 89.7 |
| Example 10 | 10 | 2.6 | 97.4 |
| Comparative Example 5 | 0 | 0 | 100 |

As shown in Table 3, in a case where the height of the liquid surface was set to 50 $\mu$m, 98% or more of the cells were circular and tetragonal cells were 2% or less. On the other hand, by decreasing the height of the liquid surface to 30 $\mu$m, 20 $\mu$m, and 10 $\mu$m, the ratio of the circular cells was decreased and the ratio of the tetragonal cells was increased. In a case where the height of the liquid surface of Comparative Example 4 is 0 $\mu$m, all of the cells were pinched with in a pore and deformed.

According to this, it was suggested that the height of the liquid surface is necessarily 30 $\mu$m or more, which enables a half or more of the cells to keep the circular shape without being deformed. In addition, in a case where the height of the liquid surface is set to 50 $\mu$m or more, it was further confirmed that it was possible to increase the ratio of the cells kept as to have a circular shape without being deformed.

<Test of extracting cells trapped on filter 1: Extraction by back wash (Discharge flow path →Introduction flow path)>

(Example 11)

A test was performed using a CTC collecting device: CT6000 (trade name manufactured by Hitachi Chemical Company, Ltd.) in which the filter of Evaluation Example 1 is set to a cartridge (corresponding to the cell trapping device according to the present embodiment). The CTC collecting device includes a flow path for introducing a blood sample or a reagent, and an inlet of the flow path is connected to a reservoir produced by processing a syringe. An operation such as trapping, staining, and washing of the CTC can be continuously and easily performed by sequentially putting the blood sample or the reagent into the reservoir.

The blood sample was introduced into the CTC collecting device to concentrate a cancer cell. A sample in which 1000 cancer cells per 1 mL of the blood are contained in the blood of a healthy person collected by an EDTA-containing vacuum blood collection tube was used as the blood sample. The aforementioned human non-small cell lung cancer cell NCI-H358 was used as the cancer cell. In addition, the blood was used after 6 hours from blood collection.

First, 1 ml of 2 mM EDTA-0.5% BSA (bovine serum albumin)-PBS (PBS manufactured by Gibco, pH 7.4) (hereinafter, washing solution) was introduced into the reservoir and filled on the filter and the filter was left standstill for 10 minutes. Subsequently, the liquid started to be sent at the flow rate of 200 $\mu$L/min using a peristaltic pump. Thereafter, 3 ml of the blood was put therein. After about 5 minutes, 9 mL of the washing solution was introduced into the reservoir to perform washing of the cells.

Next, a liquid in which 4% PFA is dissolved in the washing solution was put into the cartridge to immerse the cells for 15 minutes. After washing, a liquid in which 0.2% TritonX is dissolved in the washing solution was put into the cartridge to immerse the cells for 15 minutes.

Furthermore, after 10 minutes, the pump flow rate was changed to 20 $\mu$L/min and 600 $\mu$L of a cell staining solution (Hoechst 33342: 30 $\mu$L, Wash buffer: 300 ml) was introduced into the reservoir to fluorescent stain the cancer cells or the white blood cells on the filter. After the cells trapped on the filter were stained for 30 minutes, 1 mL of the 2mM EDTA-0.5% BSA-PBS was introduced into the reservoir to wash the cells.

Subsequently, the cancer cells and the white blood cells on the filter were photographed using a fluorescence microscope (BX61, Olympus Corporation) including a computer controlled type electric stage and a cooled digital camera (DP70, Olympus Corporation). In order to observe the fluorescent light originated from Hoechst 33342 and CellTracker Red CMTPX, an image was obtained using each of the WU and WIGfilter (Olympus Corporation). Lumina Vision (MITANI Corporation) was used for image obtainment and analysis soft. The captured image of the cells was analyzed and the number of cancer cells trapped on the filter was counted.

After all of the valves of the cartridge (corresponding to the specific cell-trapping device according to the present embodiment) in which photographing is completed were closed, a rearrangement was performed so as to drain the liquid from the discharge flow path 23 (refer to Fig. 2) to the introduction flow path 25 (refer to Fig. 2). A syringe pump TE-351 (Terumo Corporation) was connected to the discharge flow path 23 side and the liquid was sent (back wash) at the rate of 200 $\mu$L/min for 6 minutes. About 1 ml of the solution discharged from the introduction flow path was collected within a centrifugal precipitation tube.

By introducing the cell-containing solution collected by back wash into the syringe using a CTC collecting device: CT6000 (trade name manufactured by Hitachi Chemical Company, Ltd.) in which a new cartridge is set, cells on the filter were trapped again and the number of the collected cells was counted. The CTC collecting device includes a flow path for introducing a blood sample or a reagent, and an inlet of the flow path is connected to a reservoir produced by processing a syringe.

The number of the cells that had been trapped again was counted using the same fluorescence microscope.

It was calculated that the number of trapped cancer cells after back wash÷cancer cells trapped for the first time=collection rate of the cancer cells by back wash.

(Example 12)

The test was performed using the same method as Example 11 except that the rate at the time of back wash was 400 $\mu$L/min.

(Example 13)

The test was performed using the same method as Example 11 except that the rate at the time of back wash was 800 $\mu$L/min.

(Example 14)

The test was performed using the same method as Example 11 except that before the liquid is sent by back wash, the specific cell-trapping device was vibrated at 200 rpm for 5 seconds using Voltex (F202A0175) (manufactured by VELP Scientifica)

(Example 15)

The test was performed using the same method as Example 14 except that the rate at the time of back wash was 400 $\mu$L/min.

(Example 16)

The test was performed using the same method as Example 14 except that the rate at the time of back wash was 800 $\mu$L/min.

(Comparative Example 6)

The test was performed using the same method as Example 11 except that after the staining of the cells was completed, the liquid within the cell trapping device was taken out at the rate of 50 μL/min, and photographing was performed by a fluorescence microscope.

(Comparative Example 7)

The test was performed using the same method as Example 14 except that after the staining of the cells was completed, the liquid within the cell trapping device was taken out at the rate of 50 μL/min, and photographing was performed by a fluorescence microscope.

(Results)

Table 4 shows results of Example 11 to Example 16 and Comparative Examples 6 and 7.

[Table 4]

| | Presence or absence of liquid before back wash | Presence or absence of vibration before back wash | Flow rate at the time of back wash [μl/min] | Number of trapped cells before back wash [cells] | Number of trapped cells after back wash [cells] | Collection rate [%] |
|---|---|---|---|---|---|---|
| Example 11 | Presence | Absence | 200 | 1257 | 718 | 57.1 |
| Example 12 | Presence | Absence | 400 | 1113 | 854 | 76.7 |
| Example 13 | Presence | Absence | 800 | 1008 | 689 | 68.4 |
| Example 14 | Presence | Presence | 200 | 1217 | 863 | 70.9 |
| Example 15 | Presence | Presence | 400 | 1194 | 905 | 75.8 |
| Example 16 | Presence | Presence | 800 | 992 | 758 | 76.4 |
| Comparative Example 6 | Absence | Absence | 400 | 1098 | 152 | 13.8 |
| comparative Example 7 | Absence | Presence | 400 | 1167 | 322 | 27.6 |

From the results of Table 4, in a case where back wash was performed in a state where the liquid within the cell trapping device remained (Examples 11 to 16), the collection rate of the cells after back wash was 50% or more. Furthermore, in a case where vibration was imparted before back wash (Examples 14 to 16), the collection rate of the cells was enhanced than the case where vibration was not imparted. On the other hand, in a case where back wash was performed in a state where the entire liquids within the cell trapping device were taken out (Comparative Examples 6 and 7), the result was that the collection rate by back wash was low, which was 10% to 30%. According to this, in a case where back wash is performed, it was suggested that it is important to perform back wash in a state where the liquid within the cell trapping device is not taken out.

<Test of extracting cells trapped on filter 2: Extraction by back wash (Introduction flow path 1→Introduction flow path 2)>

(Example 17)

A test was performed using a CTC collecting device: CT6000 (trade name manufactured by Hitachi Chemical Company, Ltd.) in which the filter of Evaluation Example 1 is set to a cartridge (corresponding to the cell trapping device according to the present embodiment). The CTC collecting device includes a flow path for introducing a blood sample or a reagent, and an inlet of the flow path is connected to a reservoir produced by processing a syringe. An operation such as trapping, staining, and washing of the CTC can be continuously and easily performed by sequentially putting the blood sample or the reagent into the reservoir.

The blood sample was introduced into the CTC collecting device to concentrate a cancer cell. A sample in which 1000 cancer cells per 1 mL of the blood are contained in the blood of a healthy person collected by an EDTA-containing vacuum blood collection tube was used as the blood sample. The aforementioned human non-small cell lung cancer cell NCI-H358 was used as the cancer cell. In addition, the blood was used after 6 hours from blood collection.

First, 1 ml of 2 mM EDTA-0.5% BSA (bovine serum albumin)-PBS (PBS manufactured by Gibco, pH 7.4) (hereinafter, washing solution) was introduced into the reservoir and filled on the filter and the filter was left standstill for 10 minutes. Subsequently, the liquid started to be sent at the flow rate of 200 μL/min using a peristaltic pump. Thereafter, 3 ml of the blood was put therein. After about 5 minutes, 9 mL of the washing solution was introduced into the reservoir to perform washing of the cells.

Next, a liquid in which 4% PFA is dissolved in the washing solution was put into the cartridge to immerse the cells for 15 minutes. After washing, a liquid in which 0.2% TritonX is dissolved in the washing solution was put into the cartridge to immerse the cells for 15 minutes.

Furthermore, after 10 minutes, the pump flow rate was changed to 20 μL/min and 600 μL of a cell staining solution (Hoechst 33342: 30 μL, Wash buffer: 300 ml) was introduced into the reservoir to fluorescent stain the cancer cells or the white blood cells on the filter. After the cells trapped on the filter were stained for 30 minutes, 1 mL of the 2mM EDTA-0.5% BSA-PBS was introduced into the reservoir to wash the cells.

Subsequently, the cancer cells and the white blood cells on the filter were photographed using a fluorescence microscope (BX61, Olympus Corporation) including a computer controlled type electric stage and a cooled digital camera (DP70, Olympus Corporation). In order to observe the fluorescent light originated from Hoechst 33342 and CellTracker Red CMTPX, an image was obtained using each of the WU and WIGfilter (Olympus Corporation). Lumina Vision (MITANI Corporation) was used for image obtainment and analysis soft. The captured image of the cells was analyzed and the number of cancer cells trapped on the filter was counted.

After all of the valves of the cartridge (corresponding to the specific cell-trapping device according to the present embodiment) in which photographing is completed were closed, a rearrangement was performed so as to drain the liquid from the discharge flow path 23 (refer to Fig. 2) to the introduction flow path 25 (refer to Fig. 2). A syringe pump TE-351 (Terumo Corporation) was connected to the discharge flow path 23 side and the liquid was sent (back wash) at the rate of 200 μL/min for 6 minutes. About 1 ml of the solution discharged from the introduction flow path was collected within a centrifugal precipitation tube.

By introducing the cell-containing solution collected by back wash into the syringe using a CTC collecting device: CT6000 (trade name manufactured by Hitachi Chemical Company, Ltd.) in which a new cartridge is set, cells on the filter were trapped again and the number of the collected cells was counted. The CTC collecting device includes a flow path for introducing a blood sample or a reagent, and an inlet of the flow path is connected to a reservoir produced by processing a syringe.

The number of the cells that had been trapped again was counted using the same fluorescence microscope.

It was calculated that the number of trapped cancer cells after back wash÷cancer cells trapped for the first time=collection rate of the cancer cells by back wash.

(Example 18)

The test was performed using the same method as Example 18 except that the rate at the time of back wash was 400 μL/min.

(Example 19)

The test was performed using the same method as Example 18 except that the rate at the time of back wash was 800 μL/min.

(Example 20)

The test was performed using the same method as Example 18 except that before the liquid is sent by back wash, the specific cell-trapping device was vibrated at 200 rpm for 5 seconds using Voltex (F202A0175) (manufactured by VELP Scientifica)

(Example 21)

The test was performed using the same method as Example 21 except that the rate at the time of back wash was 400 μL/min.

(Example 22)

The test was performed using the same method as Example 21 except that the rate at the time of back wash was 800 μL/min.

(Comparative Example 8)

The test was performed using the same method as Example 17 except that after the staining of the cells was completed, the liquid within the cartridge was taken out at the rate of 50 μL/min, and photographing was performed by a fluorescence microscope.

(Comparative Example 9)

The test was performed using the same method as Example 20 except that after the staining of the cells was completed, the liquid within the cartridge was taken out at the rate of 50 μL/min, and p photographing was performed by a fluorescence microscope.

(Results)

Table 5 shows results of Example 17 to Example 22 and Comparative Examples 8 and 9.

[Table 5]

| | Presence or absence of liquid before back wash | Presence or absence of vibration before back wash | Flow rate at the time of back wash [μl/min] | Number of trapped cells before back wash [cells] | Number of trapped cells after back wash [cells] | Collection rate [%] |
|---|---|---|---|---|---|---|
| Example 17 | Presence | Absence | 200 | 1322 | 881 | 51.5 |
| Example 18 | Presence | Absence | 400 | 1081 | 611 | 56.5 |
| Example 19 | Presence | Absence | 800 | 892 | 665 | 74.6 |
| Example 20 | Presence | Presence | 200 | 1033 | 761 | 73.7 |
| Example 21 | Presence | Presence | 400 | 1115 | 812 | 72.8 |
| Example 22 | Presence | Presence | 800 | 908 | 680 | 74.9 |
| Comparative Example 8 | Absence | Absence | 400 | 1280 | 341 | 26.6 |
| Comparative Example 9 | Absence | Absence | 400 | 887 | 127 | 14.3 |

From the results of Table 5, in a case where back wash was performed in a state where the liquid within the cell trapping device remained (Examples 17 to 22), the collection rate of the cells after back wash was 50% or more. Furthermore, in a case where vibration was imparted before back wash (Examples 20 to 22), the collection rate of the cells was enhanced than the case where vibration was not imparted. On the other hand, in a case where back wash was performed in a state where the entire liquids within the cell trapping device were taken out (Comparative Examples 8 and 9), the result was that the collection rate by back wash was low. According to this, in a case where back wash is performed, it was suggested that it is important to perform back wash in a state where the liquid within the cell trapping device is not taken out

<Test of extracting cells trapped on filter 3: Method for collecting cells by liquid collection (suction) (open type housing)>

(Example 23)

The filter of Evaluation Example 1 was set in the housing of Fig. 1, and the liquid was sucked using a peristaltic pump (SJ-1215) (ATTO Corporation) to perform trapping of the cells on the filter. Introduction of blood was performed such that a silicon tube was connected to two syringes and the blood and the washing solution were introduced from each separate syringe using a three-way stopcock (TS-TL1K) (manufactured by Terumo Corporation).

First, 1 ml of 2 mM EDTA-0.5% BSA (bovine serum albumin)-PBS (PBS manufactured by Gibco, pH 7.4) (hereinafter, washing solution) was introduced into the reservoir and filled on the filter and the filter was left standstill for 10 minutes. Subsequently, the liquid started to be sent at the flow rate of 200 μL/min using a peristaltic pump. Thereafter, 3 ml of the blood was put therein. After about 5 minutes, 9 mL of the washing solution was introduced into the reservoir to perform washing of the cells.

The blood sample was introduced into a first syringe to concentrate a cancer cell on the filter. A sample in which 1000 cancer cells per 1 mL of the blood are contained in the blood of a healthy person collected by an EDTA-containing vacuum blood collection tube was used as the blood sample. The aforementioned human non-small cell lung cancer cell NCI-H358 was used as the cancer cell. In addition, the blood was used after 6 hours from blood collection.

Next, a liquid in which 4% PFA is dissolved in the washing solution was put into the cartridge to immerse the cells for 15 minutes. After washing, a liquid in which 0.2% TritonX is dissolved in the washing solution was put into the cartridge to immerse the cells for 15 minutes.

Furthermore, after 10 minutes, the pump flow rate was changed to 20 μL/min and 600 μL of a cell staining solution (Hoechst 33342: 30 μL, Wash buffer: 300 ml) was introduced into the reservoir to fluorescent stain the cancer cells or the white blood cells on the filter. After the cells trapped on the filter were stained for 30 minutes, 1 mL of the 2mM EDTA-0.5% BSA-PBS was introduced into the reservoir to wash the cells. A method for continuously introducing the liquid was performed without taking out the liquid at all until the final washing solution is introduced.

Subsequently, the introduction of the liquid was stopped in a state where the liquid surface was kept as the height of the frame of the housing. In this state, the liquid on the filter was collected (sucked) using a micropipette. By introducing the cell-containing solution collected by back wash into the syringe using a CTC collecting device: CT6000 (trade name manufactured by Hitachi Chemical Company, Ltd.) in which a new cartridge is set, cells on the filter were trapped again and the number of the collected cells was counted. The CTC collecting device includes a flow path for introducing a blood sample or a reagent, and an inlet of the flow path is connected to a reservoir produced by processing a syringe.

The number of the cells that had been trapped again was counted using the same fluorescence microscope.

It was calculated that the number of trapped cancer cells after back wash÷cancer cells introduced for the first time (theoretical value) =collection rate of the cancer cells by back wash.

(Comparative Example 10)

The test was performed according to the same method as Example 23, after washing was completed after the introduction of the staining solution, the liquid within the housing was completely taken out at the rate of 50 μL/min. After the liquid was completely taken out, the washing solution was introduced from the reservoir again at the rate of 200 μ/min.

Subsequently, the introduction of the liquid was stopped in a state where the liquid surface was kept as the height of the frame of the housing. In this state, the liquid on the filter was collected using a micropipette. By introducing the cell-containing solution collected by back wash into the syringe using a CTC collecting device: CT6000 (trade name manufactured by Hitachi Chemical Company, Ltd.) in which a new cartridge is set, cells on the filter were trapped again and the number of the collected cells was counted. The CTC collecting device includes a flow path for introducing a blood sample or a reagent, and an inlet of the flow path is connected to a reservoir produced by processing a syringe.

The number of the cells that had been trapped again was counted using the same fluorescence microscope.

It was calculated that the number of trapped cancer cells after back wash÷cancer cells introduced for the first time (theoretical value) =collection rate of the cancer cells by liquid collection.

(Results)

Table 6 shows results of Example 23 and Comparative Example 10.

[Table 6]

| | Presence on Absence of taking out liquid | Theoretical number of introduced cells [cells] | Number of cells trapped again after taking out liquid [cells] | Collection rate [%] |
|---|---|---|---|---|
| Example 23 | Absence | 1000 | 781 | 78.1 |
| Comparative Example 10 | Presence | 1000 | 182 | 18.2 |

From Table 6, even in the method for collecting cells by liquid collection (suction), in a case where the liquid is not taken out from the housing in the middle of the step, the collection rate of the cells were high, which was 70% or more. On the other hand, in the middle of the step of introducing the liquid, in a case where the liquid was taken out once, the collection rate of the cells were low, which was 18%. According to this, even in the method for collecting cells by liquid collection (suction), it was suggested that it is important to continuously introduce the liquid without taking out the liquid at all in the middle of the step of introducing the liquid.

**Reference Signs List**

1...MCL, 2...Peelable copper foil, 2'...Copper plate, 3...Photoresist, 3a... Photoresist exposed area (exposed area), 3b...Photoresist developed area, 4...Photomask, 5...Plated layer, 6...Through hole, 7...Gold plated layer, 10, 20...Cell trapping device, 11, 12...Filter, 12, 22...Introduction region, 13, 23...Discharge flow path, 24...Upper lid, 25, 26...Introduction flow path

**Claims**

1.   A cell trapping method for selectively trapping a specific cell included in a cell-containing solution at a filter surface by filtering a liquid using a cell trapping device, the cell trapping device including a housing which has an introduction region having a predetermined volume and for introducing a liquid selected from the cell-containing solution containing two or more types of cells and a treating solution for treating the cells in the cell-containing solution into an interior, and a discharge flow path for discharging the liquid to the exterior; and a filter which is provided on a flow path between the introduction region and the discharge flow path and in which a plurality of through holes for the liquid to flow through is formed in the thickness direction, the method comprising:

a step of draining a liquid to the cell trapping device for n (n is a natural number) times,
wherein from the first step of draining the liquid to the n-th step of draining the liquid, a liquid surface of the liquid in the introduction region on the filter is maintained at a predetermined liquid surface height, and when the n-th step of draining the liquid is completed, the discharging of the liquid from the cell trapping device is stopped in a state where the liquid surface is at a predetermined height in the filter.

2.   The cell trapping method according to Claim 1,

wherein the housing includes an upper lid disposed above the introduction region and an introduction flow path of the liquid.

3.   The cell trapping method according to Claim 1 or 2,

wherein before the first step of draining a liquid, a washing solution fills in the upstream side than the filter including the interior of the introduction region of the housing in advance.

4.   The cell trapping method according to any one of Claims 1 to 3,

wherein the step of draining a liquid includes at least one step of a step of draining a fixing solution, a step of draining a permeating solution, a step of draining a staining solution, and a step of draining a washing solution; and the step of draining a cell-containing solution.

5.   The cell trapping method according to any one of Claims 1 to 3,

wherein the n is 1 and the step of draining a liquid is the step of draining a cell-containing solution.

6.   The cell trapping method according to any one of Claims 1 to 3,

wherein in the step of draining a liquid, the liquid to be drained for the first time is a cell-containing solution, and the liquid to be drained for the second time is a washing solution.

7.   The cell trapping method according to any one of Claims 1 to 6,

wherein in the step of draining a liquid, at least part of the trapping target cell is immersed in the liquid in the introduction region on the filter.

8.  The cell trapping method according to any one of Claims 1 to 7,

    wherein in the step of draining a liquid, the height of the liquid surface of the liquid in the introduction region on the filter is equal to or greater than the size of the trapping target cell.

9.  The cell trapping method according to any one of Claims 1 to 8,

    wherein in the step of draining a liquid, the height of the liquid surface of the liquid in the introduction region on the filter is 50 μm or greater.

10. The cell trapping method according to any one of Claims 1 to 9,

    wherein a solvent of the cell-containing solution includes an aqueous solution containing whole blood, serum, or plasma of a mammal, or a protein originated therefrom.

11. The cell trapping method according to any one of Claims 1 to 10,

    wherein the filter is processed by using a polymeric material or a metal.

12. The cell trapping method according to any one of Claims 1 to 11,

    wherein in the step of draining a liquid, a rate of introducing the liquid to the cell trapping device is 50 to 1000 μL/min.

13. A method for producing a specific cell-trapping device for producing a specific cell-trapping device which selectively traps a specific cell included in a cell-containing solution at a filter surface by filtering a liquid using a cell trapping device, the cell trapping device including a housing which has an introduction region having a predetermined volume and for introducing a liquid selected from the cell-containing solution containing two or more types of cells and a treating solution for treating the cells in the cell-containing solution into an interior, and a discharge flow path for discharging the liquid to the exterior; and a filter which is provided on a flow path between the introduction region and the discharge flow path and in which a plurality of through holes for the liquid to flow through is formed in the thickness direction, the method comprising:

    a step of draining a liquid to the cell trapping device for n (n is a natural number) times,
    wherein from the first step of draining the liquid to the n-th step of draining the liquid, a liquid surface of the liquid in the introduction region on the filter is maintained at a predetermined liquid surface height, and when the n-th step of draining the liquid is completed, the discharging of the liquid from the cell trapping device is stopped in a state where the liquid surface is at a predetermined height in the filter.

14. The method for producing a specific cell-trapping device according to Claim 13,

    wherein the housing includes an upper lid disposed above the introduction region and an introduction flow path of the liquid.

15. The method for producing a specific cell-trapping device according to Claim 13 or 14,

    wherein before the first step of draining a liquid, a washing solution fills in the upstream side than the filter including the interior of the introduction region of the housing in advance.

16. The method for producing a specific cell-trapping device according to any one of Claims 13 to 15,

    wherein the step of draining a liquid includes at least one step of a step of draining a fixing solution, a step of draining a permeating solution, a step of draining a staining solution, and a step of draining a washing solution; and the step of draining a cell-containing solution.

17. The method for producing a specific cell-trapping device according to any one of Claims 13 to 15,

wherein the n is 1 and the step of draining a liquid is the step of draining a cell-containing solution.

18. The method for producing a specific cell-trapping device according to any one of Claims 13 to 15,

wherein in the step of draining a liquid, the liquid to be drained for the first time is a cell-containing solution, and the liquid to be drained for the second time is a washing solution.

19. The method for producing a specific cell-trapping device according to any one of Claims 13 to 18,

wherein in the step of draining a liquid, at least part of the trapping target cell is immersed in the liquid in the introduction region on the filter.

20. The method for producing a specific cell-trapping device according to any one of Claims 13 to 19,

wherein in the step of draining a liquid, the height of the liquid surface of the liquid in the introduction region on the filter is equal to or greater than the size of the trapping target cell.

21. The method for producing a specific cell-trapping device according to any one of Claims 13 to 20,

wherein in the step of draining a liquid, the height of the liquid surface of the liquid in the introduction region on the filter is 50 $\mu$m or greater.

22. The method for producing a specific cell-trapping device according to any one of Claims 13 to 21,

wherein a solvent of the cell-containing solution includes an aqueous solution containing whole blood, serum, or plasma of a mammal, or a protein originated therefrom.

23. The method for producing a specific cell-trapping device according to any one of Claims 13 to 22,

wherein the filter is processed by using a polymeric material or a metal.

24. The method for producing a specific cell-trapping device according to any one of Claims 13 to 23,

wherein in the step of draining a liquid, a rate of introducing the liquid to the cell trapping device is 50 to 1000 $\mu$L/min.

25. A method for producing a specific cell-containing solution obtained by selectively trapping a specific cell included in a cell-containing solution at a filter surface by filtering a liquid using a cell trapping device, the cell trapping device including a housing which has an introduction region having a predetermined volume and for introducing a liquid selected from the cell-containing solution containing two or more types of cells and a treating solution for treating the cells in the cell-containing solution into an interior, and a discharge flow path for discharging the liquid to the exterior; and a filter which is provided on a flow path between the introduction region and the discharge flow path and in which a plurality of through holes for the liquid to flow through is formed in the thickness direction, the method comprising:

a step of draining a liquid to the cell trapping device for n (n is a natural number) times, wherein from the first step of draining the liquid to the n-th step of draining the liquid, a liquid surface of the liquid in the introduction region on the filter is maintained at a predetermined liquid surface height, and when the n-th step of draining the liquid is completed, the discharging of the liquid from the cell trapping device is stopped in a state where the liquid surface is at a predetermined height in the filter, and wherein after the step of draining a liquid to the cell trapping device, a step of collecting a liquid existing in the housing is included.

26. The method for producing a specific cell-containing solution according to Claim 25,

wherein the step of collecting a liquid existing in the housing includes a step of sucking a liquid in the introduction region from the introduction region side.

**27.** The method for producing a specific cell-containing solution according to Claim 25,

wherein the housing includes an upper lid disposed above the introduction region and an introduction flow path of the liquid, and the step of collecting a liquid existing in the housing includes a step of sending a liquid from the discharge flow path to the introduction flow path.

**28.** The method for producing a specific cell-containing solution according to Claim 27,

wherein the flow rate of sending a liquid from the discharge flow path to the introduction flow path is 50 to 1000 μL/min.

**29.** The method for producing a specific cell-containing solution according to Claim 27 or 28,

wherein the housing is vibrated before a liquid is sent from the discharge flow path to the introduction flow path.

**30.** The method for producing a specific cell-containing solution according to Claim 25,

wherein the housing includes an upper lid disposed above the introduction region and two or more of the introduction flow paths of the liquid, and the step of collecting a liquid existing in the housing includes a step of sending a liquid to an introduction flow path different from a first introduction flow path among two or more introduction flow paths from the first introduction flow path included in the two or more introduction flow paths.

**31.** The method for producing a specific cell-containing solution according to Claim 30,

wherein a flow rate of sending a liquid to an introduction flow path different from a first introduction flow path among two or more introduction flow paths from the first introduction flow path included in the two or more introduction flow paths is 50 to 1000 μL/min.

**32.** The method for producing a specific cell-containing solution according to Claim 30 or 31,

wherein the housing is vibrated before a liquid is sent to an introduction flow path different from a first introduction flow path among two or more introduction flow paths from the first introduction flow path included in the two or more introduction flow paths.

**33.** The method for producing a specific cell-containing solution according to any one of Claims 25 to 32,

wherein before the first step of draining a liquid, a washing solution fills in the upstream side than the filter including the interior of the introduction region of the housing in advance.

**34.** The method for producing a specific cell-containing solution according to any one of Claims 25 to 33,

wherein the step of draining a liquid includes at least one step of a step of draining a fixing solution, a step of draining a permeating solution, a step of draining a staining solution, and a step of draining a washing solution; and the step of draining a cell-containing solution.

**35.** The method for producing a specific cell-containing solution according to any one of Claims 25 to 34,

wherein the n is 1 and the step of draining a liquid is the step of draining a cell-containing solution.

**36.** The method for producing a specific cell-containing solution according to any one of Claims 25 to 35,

wherein in the step of draining a liquid, the liquid to be drained for the first time is a cell-containing solution, and the liquid to be drained for the second time is a washing solution.

**37.** The method for producing a specific cell-containing solution according to any one of Claims 25 to 36,

wherein in the step of draining a liquid, at least part of the trapping target cell is immersed in the liquid in the introduction region on the filter.

**38.** The method for producing a specific cell-containing solution according to any one of Claims 25 to 37,

wherein in the step of draining a liquid, the height of the liquid surface of the liquid in the introduction region on the filter is equal to or greater than the size of the trapping target cell.

**39.** The method for producing a specific cell-containing solution according to any one of Claims 25 to 38,

wherein in the step of draining a liquid, the height of the liquid surface of the liquid in the introduction region on the filter is 50 $\mu$m or greater.

Fig.1

# Fig.2

*Fig.3*

(A)

(B)

[UV LIGHT]

(C)

3a    3a    3a    3a

3a 3b 3a 3b 3a 3b 3a 3b

(D)

3a    3a    3a    3a

(E)

3a    3a    3a    3a

(F)

(G)

3a    3a    3a    3a

(H)

6     6     6     6

(I)

7     7     7     7     7

# Fig.4

(A)                                                      2'

(B)                                                      3
                                                         2'

[UV LIGHT]

(C)                                                      4
                                                         3
                                                         2'

  3a      3a      3a      3a

  3a  3b  3a  3b  3a  3b  3a  3b

(D)                                                      2'

  3a      3a      3a      3a

(E)                                                      5
                                                         2'

(F)                                                      5
   3a      3a      3a      3a

(G)                                                      5
      6       6       6       6

(H)
    7       7       7       7       7

# Fig.5

LONG PORE DIAMETER

SHORT
PORE
DIAMETER

A1

*Fig.6*

*Fig.7*

*Fig.8*

10 µm

*Fig.9*

10 µm

## INTERNATIONAL SEARCH REPORT

| | International application No. |
|---|---|
| | PCT/JP2015/074457 |

A. CLASSIFICATION OF SUBJECT MATTER
*C12N1/02*(2006.01)i, *C12M1/00*(2006.01)i

According to International Patent Classification (IPC) or to both national classification and IPC

B. FIELDS SEARCHED

Minimum documentation searched (classification system followed by classification symbols)
C12N1/02, C12M1/00

Documentation searched other than minimum documentation to the extent that such documents are included in the fields searched

| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)
JSTPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| Y | JP 2014-140313 A  (Hitachi Chemical Co., Ltd.), 07 August 2014 (07.08.2014), claim 1; paragraphs [0031], [0033], [0034], [0036] to [0039]; fig. 1, 2 (Family: none) | 1-39 |
| Y | WO 2013/176106 A1  (YSEC Co., Ltd.), 28 November 2013 (28.11.2013), paragraph [0016] & JP 5545689 B | 1-39 |
| Y | JP 5-336996 A  (Koken Co., Ltd.), 21 December 1993 (21.12.1993), paragraph [0012] (Family: none) | 1-39 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30 October 2015 (30.10.15) | 10 November 2015 (10.11.15) |

| Name and mailing address of the ISA/ | Authorized officer |
|---|---|
| Japan Patent Office | |
| 3-4-3,Kasumigaseki,Chiyoda-ku, | |
| Tokyo 100-8915,Japan | Telephone No. |

Form PCT/ISA/210 (second sheet) (July 2009)

**INTERNATIONAL SEARCH REPORT**

International application No.

PCT/JP2015/074457

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2000-146782 A (Yoshio KAWAI),<br>26 May 2000 (26.05.2000),<br>paragraph [0007]<br>(Family: none) | 1-39 |
| Y | JP 2009-31300 A (Becton, Dickinson and Co.),<br>12 February 2009 (12.02.2009),<br>paragraph [0079]<br>& US 2003/0086830 A1 & WO 2003/031064 A1<br>page 22, line 9 to the last line<br>& EP 1450952 A1 | 1-39 |
| Y | JP 2005-532030 A (Becton, Dickinson and Co.),<br>27 October 2005 (27.10.2005),<br>paragraph [0065]<br>& US 2003/0087423 A1 & WO 2003/031065 A1<br>page 23, lines 7 to 20<br>& EP 1450951 A1 | 1-39 |
| Y | JP 2013-88 A (Olympus Corp.),<br>07 January 2013 (07.01.2013),<br>paragraphs [0017] to [0019]<br>(Family: none) | 27-39 |
| Y | JP 2008-54521 A (Canon Inc.),<br>13 March 2008 (13.03.2008),<br>paragraphs [0092], [0101], [0102]; fig. 7-1,<br>7-2<br>& US 2008/0057561 A1<br>paragraphs [0138], [0147], [0148]; fig. 7A, 7B | 30-39 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010135603 A

- JP 2013042689 A